# EUROPÄISCHE PATENTSCHRIFT

(11) **EP 1 973 929 B1**
(45) Veröffentlichungstag und Bekanntmachung des Hinweises auf die Patenterteilung: **29.09.2010**
(21) Anmeldenummer: 07703050.0
(22) Anmeldetag: 26.01.2007
(51) Int. Cl.: C07K 5/068, C07K 5/06, A61L 33/04, G01N 33/49, G01N 33/52

(54) **ANTIKOAGULATION VON HUMANEM BLUT EX VIVO**
ANTICOAGULATION OF HUMAN BLOOD EX VIVO
ANTICOAGULATION DU SANG HUMAIN EX VIVO

(30) Priorität: 26.01.2006 DE 102006003677; 12.10.2006 DE 102006048300
(43) Veröffentlichungstag der Anmeldung: 01.10.2008
(73) Patentinhaber: Hellstern, Peter, 67240 Bobenheim-Roxheim (DE); Stürzebecher, Uta, 99094 Erfurt-Rhoda (DE)
(72) Erfinder: HELLSTERN, Peter, 67240 Bobenheim-Roxheim (DE); STÜRZEBECHER, Jörg, deceased (DE)
(74) Vertreter: Luderschmidt, Schüler & Partner
(86) Internationale Anmeldenummer: PCT/EP2007/000661
(87) Internationale Veröffentlichungsnummer: WO 2007/085461

(56) Entgegenhaltungen:
- EP-A- 1 217 000
- WO-A-02/46159
- WO-A-97/24118
- WO-A-98/49563
- WO-A-03/076457
- US-A- 5 633 381
- HINDER MARKUS ET AL: "ANTICOAGULANT AND ANTI-PLATELET EFFECTS ARE MAINTAINED FOLLOWING COADMINISTRATION OF OTAMIXABAN, A DIRECT FACTOR XA INHIBITOR, WITH TIROFIBAN IN HEALTHY VOLUNTEERS" THROMBOSIS AND HAEMOSTASIS, STUTTGART, DE, Bd. 93, Nr. 4, April 2005 (2005-04), Seiten 794-795, XP008078339 ISSN: 0340-6245
- SAIAH E ET AL: "SMALL MOLECULE COAGULATION CASCADE INHIBITORS IN THE CLINIC" CURRENT TOPICS IN MEDICINAL CHEMISTRY, BENTHAM SCIENCE PUBLISHERS, HILVERSUM, NL, Bd. 5, Nr. 16, 2005, Seiten 1677-1895, XP009076310 ISSN: 1568-0266

## Beschreibung

Die vorliegende Erfindung betrifft allgemein ein Verfahren zur Antikoagulation von humanem Blut ex vivo und die Verwendung des antikoagulierten Bluts für die Diagnostik von zellulären Blutbestandteilen, wie zum Beispiel Thrombozyten. Außerdem betrifft die vorliegende Erfindung einen Kit für die Diagnostik von zellulären Blutbestandteilen. Die vorliegende Erfindung betrifft ferner einen Inhibitor des Blutgerinnungsfaktors Xa und die Verwendung dieses Inhibitors zur Antikoagulation von humanem Blut ex vivo. Weiterhin betrifft die Erfindung ein Verfahren zur Antikoagulation von humanem Blut ex vivo, bei dem dieser Inhibitor verwendet wird. Außerdem betrifft die Erfindung die Verwendung des Inhibitors des Blutgerinnungsfaktors Xa zur Antikoagulation von humanem Blut ex vivo für diagnostische Zwecke bzw. für die Haltbarmachung und Lagerung von Blutprodukten, wie Thrombozytapheresekonzentraten. Darüber hinaus betrifft die vorliegende Erfindung ein diesen Inhibitor enthaltendes Röhrchen zur Entnahme von Blutproben, ein diesen Inhibitor enthaltendes Aphereschlauch-/beutelsystem sowie ein diesen Inhibitor enthaltendes Thrombozytenkonzentrat. Ferner betrifft die vorliegende Erfindung einen Kit für die Diagnostik von zellulären Blutbestandteilen, wie zum Beispiel Thrombozyten.

Der menschliche Körper erleidet Tag für Tag Hunderte meist kleinere, manchmal aber auch größere, innerliche und äußerliche Verletzungen. Damit diese Verletzungen ohne größere Folgen für die Gesundheit bleiben, sorgt das Blutgerinnungssystem dafür, dass es im Fall einer Verletzung zu einem Wundverschluss kommt und das Blut nach dem Austritt aus dem Blutgefäß erstarrt. Dieses Blutgerinnungssystem besteht im Wesentlichen aus zwei Komponenten: der primären Blutstillung, die auf die Funktion der Thrombozyten zurückzuführen ist, und der plasmatischen Gerinnung, die das Zusammenspiel von einer Vielzahl von Blutgerinnungsfaktoren erfordert. Allerdings kann das Blutgerinnungssystem bei vielen Menschen aufgrund von erworbenen oder angeborenen Thrombozytenfunktionsstörungen verändert sein. Dies hat Blutungsrisiken unterschiedlichster Ausprägungen zur Folge und kann spontan oder perioperativ zu akut lebensbedrohlichen Blutgerinnungsstörungen führen.

Aus diesem Grund ist es gerade vor operativen oder invasiven Eingriffen notwendig, an vom Patienten stammenden Blutproben die Funktionstüchtigkeit der Thrombozyten zu überprüfen. Allerdings bereitet dabei die plasmatische Gerinnung des Blutes (Fibrinbildungssystem) in der Probe außerordentlich große Schwierigkeiten. Diese führt dazu, dass das Blut nach der Abnahme nur für kurze Zeit vollständig ungeronnen bleibt, und über die Bildung von Thrombin eine rasche Aggregation der Blutplättchen (Thrombozyten) induziert wird. In einer Probe mit aktivierten bzw. aggregierten Thrombozyten lassen sich jedoch keine zuverlässigen Thrombozytenfunktionstests mehr durchführen.

Die Blutgerinnung basiert auf einer kaskadenartigen Reaktionsfolge. Dabei werden inaktive Proenzyme proteolytisch in aktive Proteasen umgewandelt, die wiederum das in der Kaskade folgende Proenzym aktivieren. Prinzipiell kann die Blutgerinnung auf zwei anfänglich verschiedenen Wegen verlaufen, dem intrinsischen Weg und dem extrinsischen Weg.

Beim intrinsischen Weg werden ausgehend von der Protease Kallikrein zunächst die Blutgerinnungsfaktoren XII, XI und IX aktiviert. In einer von der Gegenwart von Calciumionen, anionischen Phospholipiden und dem aktivierten Blutgerinnungsfaktor VIIIa abhängigen Reaktion erfolgt darauf hin die Aktivierung des Blutgerinnungsfaktors Xa. Auch der extrinsische Weg führt über Thromboplastin und den Blutgerinnungsfaktor VIIa zum aktivierten Blutgerinnungsfaktor Xa. Dieser ist bei Anwesenheit von Calciumionen, anionischen Phospholipiden und dem Blutgerinnungsfaktor Va in der Lage, die Bildung von Thrombin (Blutgerinnungsfaktor IIa) aus dem Proenzym Prothrombin zu katalysieren. Thrombin bewirkt schließlich, dass das lösliche Plasmaprotein Fibrinogen in polymerisierbares Fibrin umgewandelt wird und dieses sich als faserartiges Netzwerk im Primärthrombus ablagert.

Thrombin ist allerdings nicht nur für die Bildung von Fibrin verantwortlich, sondern stellt einen starken Aktivator der Thrombozyten dar. Dabei bindet das in der Blutgerinnungskaskade gebildete Thrombin an den hochaffinen Thrombin-Rezeptor der Thrombozyten, wodurch schließlich eine Formveränderung (shape change) der Thrombozyten, gefolgt von deren Aggregation und Freisetzung prokoagulatorischer Inhaltsstoffe, induziert wird.

Aus dem Stand der Technik sind mehrere Verfahren bekannt, mit denen sich wirksam eine Verhinderung der Koagulation, also eine Antikoagulation erreichen lässt. Standardmäßig wird dabei Citrat bzw. EDTA eingesetzt. Aufgrund der Komplexierung der Calciumionen wird dabei die Aktivierung der Blutgerinnungsfaktoren VII, IX, X und Prothrombin zunächst völlig unterbunden, so dass es im Blut zu keiner Gerinnung kommt, d. h. es wird weder Fibrin aus Fibrinogen gebildet noch eine Aggregation der Thrombozyten induziert. Nach einiger Zeit wird die Antikoagulation durch Aktivierung des Gerinnungssystems über die Calcium-unabhängigen Faktoren Kallikrein, XII und XI teilweise überspielt, sodass sich letztlich Spuren von Thrombin bilden. Während die Gegenwart von Citrat oder EDTA keinen direkten Einfluss auf die verschiedenen Komponenten des Gerinnungssystems hat, wirkt sich der Entzug von Calciumionen negativ auf die im Blut befindlichen zellulären Bestandteile aus. Jede lebende Zelle benötigt einen intrazellulären Calciumspiegel zur Aufrechterhaltung ihrer normalen Funktionalität. Durch den fortschreitenden Entzug des Calciums nach der Antikoagulation von Blut mittels Citrat oder EDTA kommt es zunächst zur Veränderung bzw. Einschränkung der normalen Funktion der Zellen, was schließlich zum Absterben der Zellen führen kann. Dies betrifft insbesondere die Thrombozyten, gilt aber ebenso für Leukozyten und Erythrozyten.

So zeigt sich bei Thrombozyten sehr deutlich, dass diagnostische Funktionstests in Citrat- bzw. EDTA-Blut/Plasma nur über wenige Stunden nach der Blutabnahme zuverlässige Ergebnisse liefern und diese Ergebnisse nicht die in vivo Verhältnisse widerspiegeln, wo Calciumionen anwesend sind.

Als alternative Antikoagulanzien, die nicht über die Komplexierung von Calcium ihre Wirkung entfalten, werden Heparin und Hirudin eingesetzt. Der Hemmeffekt von Heparin auf das Blutgerinnungssystem beruht auf der Bindung an Antithrombin III. Antithrombin III inaktiviert in hohem Maße irreversibel Serinproteasen, wie Thrombin und weitere Blutgerinnungsfaktoren wie XIIa, XIa, Xa und IXa. Die Interaktion von Antithrombin III mit Heparin führt zur einer Erhöhung der Geschwindigkeit dieser durch Antithrombin III bedingten Reaktionen und unterbindet somit wirksam die Koagulation. Allerdings besitzt Heparin eine hohe Affinität zu bestimmten, für die Aktivierung von Thrombozyten notwendigen Rezeptoren, was dazu führt, dass mit Heparin behandeltes Blut zum Beispiel nicht für Thrombozytenfunktionstests eingesetzt werden kann.

Hirudin ist ein hochaffiner, natürlicher Inhibitor des Fibrinogen-spaltenden Gerinnüngsenzyms Thrombin. Tatsächlich ist eine ausreichende Hirudin-Konzentration in der Lage, humanes Blut über einen längeren Zeitraum ungeronnen zu halten. Da allerdings Hirudin ausschließlich Thrombin hemmt und damit die Fibrin-Bildung unterdrückt, kommt es zunehmend zur Aktivierung der Gerinnungskaskade bis hin zur Thrombin-Bildung. Obwohl das gebildete Thrombin zum größten Teil durch das Hirudin gebunden wird, ist trotzdem ein Teil in der Lage, an den hochaffinen Thrombin-Rezeptor der Thrombozyten zu binden. Dies führt zur Aktivierung der Thrombozyten, indem diese ihre Form wandeln, aggregieren und Inhaltsstoffe freisetzen. Es hat sich gezeigt, dass mit einer Antikoagulation des Blutes durch Hirudin keine Verbesserung der Diagnostik der Thrombozytenfunktion möglich ist.

Für die derzeit üblichen Verfahren zur Thrombozytenfunktionsdiagnostik wird Citrat-Vollblut oder plättchenreiches Plasma (PRP) aus Citrat-Vollblut verwendet. Da Thrombozyten für ihre normale Funktionstüchtigkeit Calciumionen benötigen, spiegeln die Ergebnisse der Thrombozytenfunktionstests in Citratvollblut nicht die Verhältnisse in vivo wider. Dieser Umstand steht einer breiteren Anwendung von Thrombozytenfunktionstests sehr im Wege. Gelänge es, klinisch aussagekräftige Funktionstests reproduzierbar im physiologischen Milieu durchzuführen, würde vor jedem operativen oder invasiven Eingriff ein Screeningtest auf die Thrombozytenfunktion hin durchgeführt werden, um die häufigen Thrombozytenfunktionsstörungen (Thrombozytopathien) sicher zu erfassen. Weiterhin würde die Implementierung von geeigneten Thrombozytenfunktionstests zur Erkennung einer Thrombozytenüberfunktion als Risikofaktor für Herzinfarkt und

Schlaganfall sowie zur Überwachung einer Therapie mit Thrombozytenfunktionshemmern (zum Beispiel Aspirin, Clopidigrel, parenterale verabreichte Thrombozytenfunktionshemmer) erheblich erleichtert.

Der Erfindung liegt daher die Aufgabe zu Grunde, ein neues Verfahren zur Antikoagulation von humanem Blut zu finden, wobei weder die Calcium-Konzentration im Blut verändert wird, noch eine Bildung von Thrombin erfolgt, und wobei die Funktion der Thrombozyten nicht beeinflusst wird, so dass das derart antikoagulierte Blut für die Diagnostik von zellulären Blutbestandteilen, wie zum Beispiel Thrombozyten, eingesetzt werden kann. Eine weitere Aufgabe war es, eine Verbindung zu finden, die die erfolgreiche Durchführung dieses Verfahrens ermöglicht.

Überraschend wurde nun gefunden, dass humanes Blut über einen längeren Zeitraum ungeronnen bleibt, ohne dass es zur Bildung von Thrombin oder zur Beeinflussung der Thrombozytenfunktion kommt, wenn die Blutgerinnungskaskade durch einen Hemmstoff des Blutgerinnungsfaktors Xa gemäß Formel (I) unterbrochen wird.

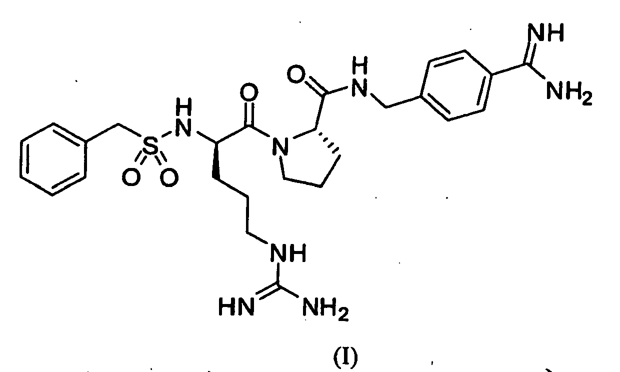

Die Verbindung gemäß Formel (I) wird im Folgenden auch als Benzylsulfonyl-D-Argininyl-Prolyl-4-Amidinobenzylamid oder Kurz BAPA' bezeichnet.

Erfindungsgemäß wird ein Verfahren zur Verfügung gestellt, mit dem sich die Antikoagulation von humanem Blut ex vivo erreichen lässt und das es erlaubt, das antikoagulierte Blut für die Diagnostik von zellulären Blutbestandteilen, wie zum Beispiel für Thrombozytenfunktionstests, einzusetzen. Dieses Verfahren ist dadurch gekennzeichnet, dass dem entnommenen Blut wenigstens ein Agens mit direkter Inhibitorwirkung auf den Blutgerinnungsfaktor Xa gemäß Formel (I) zugesetzt wird. Weiterhin betrifft die Erfindung ein Röhrchen zur Entnahme von Blutproben, ein Aphereseschlauch-/beutelsystem und ein Thrombozytenkonzentrat, die diese Verbindung enthalten.

Im Sinne der Erfindung bedeutet Antikoagulation, dass die Neubildung von Thrombin in dem entnommenen Blut inhibiert wird. Erfindungsgemäß wird die Thrombin-Neubildung inhibiert, wenn im entnommenen Blut nicht mehr als 1 nM Thrombin neu gebildet wird. Wie oben beschrieben, stellt die Aktivierung von Prothrombin zu Thrombin einen wesentlichen Schritt in der Blutgerinnungskaskade dar. Dabei wird das Prothrombinfragment 1+2 (F1+2) in äquivalenten Mengen freigesetzt. Somit wird durch die Bestimmung des entstandenen Fragments die Quantifizierung des tatsächlich entstandenen Thrombins möglich, wodurch das Prothrombinfragment F1+2 als indirekter Marker einer Aktivierung von Prothrombin zu Thrombin dienen kann. Die Konzentration des gebildeten Prothrombinfragments F1+2 kann zum Beispiel immunologisch mit kommerziellen Enzymimmunoassays (zum Beispiel Enzygnost F1+2 micro, Dade Behring GmbH, Marburg) bestimmt werden.

Erfindungsgemäß bedeutet Antikoagulation also, dass nicht mehr als 1 nM Prothrombinfragment F1+2 im Blut nach dessen Entnahme neu gebildet wird. Beträgt zum Beispiel die anfängliche Konzentration des Prothrombinfragments F1+2 in der Blutprobe unmittelbar nach der Blutentnahme 0,3 nM, dann ist das in der Probe vorhandene Blut im Sinn der Erfindung solange antikoaguliert, bis die Gesamtkonzentration des Prothrombinfragments F1+2 in der Blutprobe in diesem Beispiel 1,3 nM nicht überschreitet. Ohne geeignete Behandlung des Bluts zur Verhinderung der Koagulation kommt es aufgrund der plasmatischen Gerinnung innerhalb von wenigen Minuten zu einer Zunahme der Konzentration des Prothrombinfragments F1+2 in diesem Beispiel auf weit über 1,3 nM, d. h. das Blut koaguliert. Durch das erfindungsgemäße Verfahren wird erreicht, dass die Konzentration des Prothrombinfragments F1+2 in diesem Beispiel über einen langen Zeitraum (zum Beispiel über 48 Stunden) 1,3 nM nicht übersteigt, d. h. das Blut ist über diesen Zeitraum antikoaguliert.

Die Dauer, mit der unter Verwendung des erfindungsgemäßen Verfahrens die Antikoagulation von humanem Blut ex vivo aufrecht erhalten werden kann, d. h. der Zeitraum von der Blutentnahme bis zu dem Zeitpunkt, zu dem mehr als 1 nM Thrombin neu gebildet worden ist, beträgt mehr als 8 Stunden, vorzugsweise mehr als 12 Stunden und noch bevorzugter mehr als 24 Stunden.

Aufgrund der Inhibierung der Thrombin-Bildung wird schließlich auch die durch Thrombin induzierte Aktivierung der Thrombozyten weitgehend verhindert. Bei der Aktivierung der Thrombozyten kommt es zur Freisetzung von Thrombozyten-Inhaltsstoffen (zum Beispiel Plättchenfaktor 4 (PF 4), beta-Thromboglobulin, Serotonin, ADP/ATP und Mikropartikeln) aus den Thrombozytengranula in das Plasma und zur Expression von Aktivierungsmarkern an der Thrombozytenoberfläche (zum Beispiel P-Selectin, Thrombospondin, Multimerin, DC63, LAMP-1, PAC1 oder 9F9). Durch die Messung der Konzentration dieser Thrombozyten-Inhaltsstoffe bzw. dieser Aktivierungsmarker (zum Beispiel mittels Testkits, wie beispielhaft für PF4: ELISA PF4, Haemochrom Diagnostica GmbH, Essen) kann die Inhibierung der Aktivierung der Thrombozyten durch Hemmung der Thrombinbildung nachgewiesen werden.

Der hierin verwendete Begriff "Blut" umfasst nicht nur Vollblut, sondern auch allgemein zur Koagulation befähigtes Blut in reiner, verdünnter oder konzentrierter Form, wobei das Blut seine natürliche Zusammensetzung aufweisen oder auch von dieser abweichen kann. Der Begriff Blut kann also zum Beispiel auch plättchenreiches Plasma (PRP) oder andere Blutprodukte, wie Thrombozytenkonzentrate oder Thrombozytapheresekonzentrate umfassen.

Das für das Verfahren gemäß der vorliegenden Erfindung verwendete humane Blut kann auf jede, dem Fachmann bekannte Art und Weise entnommen werden. Bevorzugt erfolgt die Entnahme des Blutes durch Venenpunktion.

Erfindungsgemäß verwendet bezieht sich "ex vivo" auf Vorgänge außerhalb des menschlichen oder tierischen Organismus. Im Sinne der Erfindung umfasst "ex vivo" auch die Bedeutung von "in vitro". Somit bezieht sich "ex vivo" sowohl auf Untersuchungen an Proben bei noch bestehender Verbindung zwischen Probe und Mensch oder Tier, als auch auf Untersuchungen, bei denen diese Verbindung nicht mehr besteht. Ausdrücklich ausgenommen von der Bezeichnung ex vivo sind Vorgänge, die sich im menschlichen oder tierischen Körper abspielen.

Erfindungsgemäß wird die Antikoagulation von humanem Blut ex vivo erreicht, wenn dem entnommenen Blut wenigstens ein Agens mit direkter Inhibitorwirkung auf den Blutgerinnungsfaktor Xa gemäß Formel (I) zugesetzt wird.

Direkte Inhibitorwirkung bedeutet hierbei, dass es zu einer unmittelbaren Interaktion zwischen dem Inhibitor einerseits und dem zu inhibierenden Molekül andererseits kommt. Im Gegensatz dazu ist die Hemmwirkung eines indirekten Inhibitors zum Beispiel darauf zurückzuführen, dass dieser an einen direkten Inhibitor bindet und dessen Hemmwirkung verstärkt. Heparin ist demnach zum Beispiel ein indirekter Inhibitor von Blutgerinnungsfaktoren, da Heparin Antithrombin III bindet und dessen inhibitorische Wirksamkeit potenziert, nicht aber direkt mit den zu inhibierenden Blutgerinnungsfaktoren interagiert. Ein weiteres Beispiel für einen indirekten Inhibitor ist ein Inhibitor, der nicht das zu inhibierende Molekül selbst bindet, sondern mit einem Cofaktor dieses Moleküls interagiert und so dessen Funktion verhindert.

Direkte Inhibitoren des Blutgerinnungsfaktors Xa sind demnach Inhibitoren, die eine direkte Interaktion mit dem Blutgerinnungsfaktor Xa eingehen und diesen somit inaktivieren. Ein Inhibitor, der ein Enzym oder mehrere Enzyme inhibiert, die in der Blutgerinnungskaskade vor dem Blutgerinnungsfaktor Xa stehen, wie zum Beispiel die Blutgerinnungsfaktoren XIIa, XIa, IXa oder VIIa, aber nicht mit dem Blutgerinnungsfaktor Xa interagiert, um dessen Funktion in der Blutgerinnungskaskade zu inhibieren, ist demnach ein indirekter Inhibitor des Blutgerinnungsfaktors Xa. Andererseits kann ein direkter Inhibitor des Blutgerinnungsfaktors Xa auch ein Inhibitor sein, der den Blutgerinnungsfaktor Xa direkt inhibiert, und zusätzlich weitere, zum Beispiel in der Blutgerinnungskaskade vor dem Blutgerinnungsfaktor Xa liegende Enzyme inhibiert.

Erfindungsgemäß ist es wesentlich, dass das Agens gemäß Formel (I) eine direkte Inhibitorwirkung auf den Blutgerinnungsfaktor Xa ausübt und zugleich die Aktivierbarkeit der Thrombozyten nicht beeinträchtigt.

Der Stand der Technik kennt eine Vielzahl von direkten Inhibitoren des Blutgerinnungsfaktors Xa (Übersichtsartikel: Quan und Smallheer, Curr. Opin. In Drug Discovery & Development 7, 460, 2004; Pauls et al., Frontiers in Medicinal Chemistry - Online 1, 129, 2004; Maignan und Mikol, Curr. Topics in Med. Chemistry 1, 161, 2001). Obwohl sich diese nicht auf wenige Stoffklassen beschränken lassen, können einige von ihnen bestimmten Stoffklasse zugeordnet werden. Diese Stoffklassen umfassen zum Beispiel Benzamidin-Derivate (Song et al., Bioorg. Med. Chem. Lett. 13, 297, 2003), Bis-Benzamidine, Benzylamin-Derivate, Aminobenzisoxazol-Derivate, Naphthamidin-Derivate, Ketopiperazine-Derivate, 5-Chlorthiophen-Derivate, verschiedene Verbindungen mit bizyklischen Kernen, Triazol-Derivate (Deng et al. 226th ACS National Meeting New York, USA, MEDI 80, 2003), Chlorindol-Derivate (Sheehan et al., Bioorg. Med. Chem. Lett. 12, 2043, 2002), Chlomapthyl-Derivate (Jia et al., Bioorg. Med. Chem. Lett. 14, 1229, 2004), Chlorpyridyl-Derivate (Zhang et al., Bioorg. Med. Chem. Lett. 14, 989, 2004), 1-Aminoisochinolin-Derivate (Song et al., Bioorg. Med. Chem. Lett. 14, 1229, 2004), Benzoxazinon-Derivate oder acylierte 4-Amidinobenzylamine (WO 2004/062657).

Beispiele für Inhibitoren umfassen BABCH (Pefabloc tPA/Xa; Stürzebecher et al., Thromb. Res. 54, 245, 1989), ZK 807834 (Berlex Biosciences, Shaw et al., J. Med. Chem. 41, 3551, 1998), FXV 673 (Otamixaban, Sanofi-Aventis; Guertin et al., Bioorg. Med. Chem. Lett. 12, 1671, 2002), DPC 423 und DPC 602 (jeweils von Bristol-Myers-Squibb; Quan und Wexler, Curr. Topics Med. Chem. 1, 137, 2001), DPC 906 (Razaxaban; Batt et al., Bioorg. Med. Chem. Lett. 14, 5269, 2004), DX-9065a (Daiichi; Katakura et al., Biochem. Biophys. Res. Chem. 197, 965, 1993), YM-60828 (Yamanouchi Pharmaceutical Co.; Hirayama et al., Bioorg. Med. Chem. 11, 367, 2003), RPR 209685 (Sanofi-Aventis; Choi-Sledeski et al., J. Med. Chem. 46, 68, 2003), BAY 59-7939 (Bayer; Perzbom et al., J. Thromb. Haemost. 3, 514, 2005), EMD 495235 (Merck Darmstadt; Mederski et al., Bioorg. Med. Chem. Lett. 14, 3763, 2004; Mederski et al., Bioorg. Med. Chem. Lett. 14, 5817, 2004), PD-198961 (Pfizer; Willardsen et al., J. Med. Chem. 47, 4089, 2004), Pefabloc tPA/Xa (Pentapharm Ltd. Basel, Schweiz) und CJ-FXa (Haemochrom Diagnostica GmbH, Essen).

Der hierin beschriebene Inhibitor des Blutgerinnungsfaktors Xa kann gemäß der Erfindung auch in der Form von Salzen, bevorzugt physiologisch akzeptablen Salzen vorliegen. Beispielsweise kann die Verbindung als Salz mit Säuren, wie z. B. Mineralsäuren oder geeigneten organischen Säuren vorliegen. Bevorzugt liegt der erfindungsgemäße Inhibitor des Blutgerinnungsfaktors Xa gemäß Formel (I) als Salz der Trifluoressigsäure (TFA) vor.

Grab vereinfacht weist der erfindungsgemäße Inhibitor des Blutgerinnungsfaktors Xa gemäß Formel (I) die Struktur P₄-P₃-P₂-P₁ mit den folgenden vier Bausteinen auf:
P1: Ein 4-Amidinobenzylaminrest gemäß 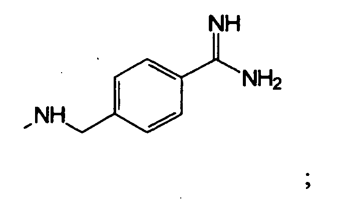
P2: Ein L-Prolylrest gemäß 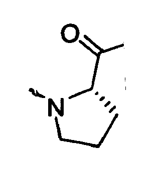 P3: Ein D-Argininylrest gemäß 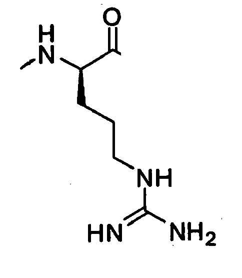
P4: Ein Benzylsulfonylrest
   gemäß

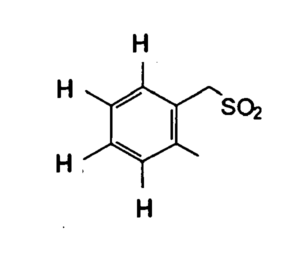

Die Darstellung des erfindungsgemäßen Inhibitors des Blutgerinnungsfaktors Xa kann durch die Anwendung oder Abwandlung von dem Fachmann bekannten Verfahren erfolgen, wobei darunter zum Beispiel in der Literatur beschriebene Verfahren zu verstehen sind. Zum Beispiel kann die Synthese in Analogie zu der in der Literatur beschriebenen Synthese von Benzylsulfonyl-D-Arg-Gly-4-Amidinobenzylamid erfolgen (Schweinitz et al., Medicinal Chemistry 2, 349-361, 2006). Dazu werden zunächst die zwei Blöcke entsprechend P4-P3-OH und H-P2-P1 synthetisiert. Die Synthese des Blockes P4-P3-OH, Benzylsulfonyl-D-Arg(Pbf)-OH kann gemäß Schweinitz et al. (Medicinal Chemistry 2, 349-361, 2006) erfolgen. Der Block H-P2-P1, H-Pro-4-Amidinobenzylamid x 2 HCl, kann gemäß Steinmetzer und Nowak (WO 02/059065) synthetisiert werden. Dabei wird das H-P2-4-Amidinobenzylamid-Derivat durch katalytische Hydrierung (mit Pd/C) aus dem entsprechenden Cbz-P2-4-Acetylhydroxyamidinobenzylamid hergestellt, das durch Kopplung der Cbz (Carbobenzyloxy) -geschützten P2-Aminosäure und H-4-Acetylhydroxyamidinobenzylamin erhalten werden kann.
Die Kopplung der Blöcke kann zum Beispiel mittels HBTU (2-(1*H*-Benzotriazol-1-yl)-1,1,3,3-tetramethyluronium Hexafluorophosphat) oder PyBop (Benzotriazol-1-yloxytripyrrolidinophosphonium Hexafluorophosphat) und Diisopropylethylamin in DMF (Arg in P3 mit anschließender Abspaltung der Pbf-Schutzgruppe mittels Trifluoressigsäure) erfolgen. Dem Fachmann ist klar, dass die Synthese des erfindungsgemäßen Inhibitors des Blutgerinnungsfaktors Xa gemäß Formel (I) selbstverständlich auch auf einem anderen als dem vorstehend beschriebenen Weg erfolgen kann, beispielsweise unter Verwendung eines P2-P1-Bausteins, bei dem die Amidinogruppe als Acetylhydroxyamidin oder als Cbz-geschütztes Amidin vorliegt (Schweinitz et al., J. Biol. Chem. 279, 33613-33622, 2004).

In einer bevorzugten Ausführungsform des erfindungsgemäßen Verfahrens erfolgt die direkte Inhibierung des Blutgerinnungsfaktors Xa kompetitiv, wobei der kompetitive Inhibitor mit dem zu hemmenden Blutgerinnungsfaktor einen stöchiometrischen, reversiblen Komplex bildet. Dies ist bspw. auch bei dem erfindungsgemäßen Inhibitor gemäß Formel (I) der Fall.

Besonders vorteilhaft ist, dass der erfindungsgemäße Inhibitor des Blutgerinnungsfaktors Xa eine starke Inhibitorwirkung gegenüber dem Blutgerinnungsfaktor Xa aufweist. Für das erfindungsgemäße Verfahren sind insbesondere solche Inhibitoren des Blutgerinnungsfaktors Xa vorteilhaft, die eine direkte Inhibitorwirkung gegenüber dem Blutgerinnungsfaktor Xa mit Kᵢ < 10 nM, vorzugsweise < 5 nM, insbesondere < 3 nM erzielen, wobei Kᵢ für die Dissoziationskonstante des Enzym-Inhibitor-Komplexes steht, also für den Quotienten aus dem Produkt der Konzentrationen von Enzym und Inhibitor und der Konzentration des Enzym-Inhibitor-Komplexes. Starke Inhibitoren bilden demnach stabile Enzym-Inhibitor-Komplexe und führen zu kleineren Kᵢ-Werten als dies bei schwächeren Inhibitoren der Fall ist. Die Bestimmung des Kᵢ-Wertes erfolgt bevorzugt durch die Messung der Enzymaktivität bei wenigstens zwei Substratkonzentrationen und jeweils wenigstens drei unterschiedlichen Inhibitor-Konzentrationen. Das Verfahren zur Bestimmung der Kᵢ-Werte erfolgt bei 25°C in Tris-Puffer (0,05 mol/l, pH 8,0, enthaltend 0,154 mol/l NaCl und 5% Ethanol). Dazu werden 200 µl Inhibitor-Lösung (gelöst im oben genannten Tris-Puffer) mit 25 µl Substrat (CH₃OCO-D-Cha-Gly-Arg-pNA; mit Cha = Cyclohexylalanin und pNA = para-Nitroanilin; vertrieben von Pentapharm AG, Basel, Schweiz unter dem Handelsnamen Pefachrome FXa; gelöst in H₂O) gemischt und die Reaktion durch Zugabe von 50 µl Enzym (Blutgerinnungsfaktor Xa, zum Beispiel von Haemochrom Diagnostica GmbH, Essen; gelöst in 0,154 mol/l NaCl) gestartet. Nach einer Reaktionszeit von 3 - 5 Minuten wird Essigsäure (25 µl) zugegeben und die Extinktion der Lösung bei 405 nm mit einem Microplate Reader (zum Beispiel iEMS Reader MF 1401, Labsystems, Helsinki, Finnland) gemessen. Die Auswertung erfolgt graphisch gemäß der Methode von Dixon (Biochem J. 55, 170, 1953).

Der direkte Inhibitor des Blutgerinnungsfaktors Xa gemäß Formel (I) inhibiert zusätzlich wenigstens ein weiteres Enzym der Frühphase der Blutgerinnung, wie zum Beispiel Plasma-Kallikrein und/oder die Blutgerinnungsfaktoren XIa, XIIa und/oder VIIa. Dadurch kann die beginnende Blutgerinnungsaktivierung schon auf einer frühen Stufe beeinflusst werden.

Die erfindungsgemäße Verbindung gemäß Formel (I) ist überraschenderweise in der Lage, neben dem Blutgerinnungsfaktor Xa insbesondere Thrombin und Plasma-Kallikrein zu hemmen. Die Bestimmung der Kᵢ-Werte des erfindungsgemäßen Inhibitors gegenüber Thrombin und Plasma-Kallikrein erfolgt bevorzugt analog zu der oben beschriebenen Bestimmung der Kᵢ-Werte des Inhibitors gegenüber dem Blutgerinnungsfaktor Xa, wobei jedoch als Substrate CH₃SO₂-D-HHT-Gly-Arg-pNA (mit HHT = Hexahydrotyrosin, und pNA = para-Nitroanilin; vertrieben unter dem Handelsnamen Pefachrome tPA) im Fall von Thrombin bzw. Bz-Pro-Phe-Arg-pNA (mit Bz = Benzyl-, und pNA = para-Nitroanilin; vertrieben unter dem Handelsnamen Chromozym PK) im Fall von Plasma-Kallikrein eingesetzt werden.

Es soll an dieser Stelle ausdrücklich angemerkt werden, dass sich die Antikoagulation von humanem Blut ex vivo auch erreichen lässt, wenn dem entnommenen Blut wenigstens ein Agens mit direkter Inhibitorwirkung auf einen anderen Blutgerinnungsfaktor als Xa zugesetzt wird. So kann humanes Blut ex vivo auch antikoaguliert werden, wenn diesem kein direkter Inhibitor des Blutgerinnungsfaktors Xa, sondern wenigstens ein Inhibitor von anderen, zum Beispiel in der Blutgerinnungskaskade vor dem Blutgerinnungsfaktor Xa stehenden Blutgerinnungsfaktoren (zum Beispiel Plasma-Kallikrein) zugesetzt wird. Allerdings führen diese Inhibitoren in der Regel erst in relativ hohen Konzentrationen zum Erfolg, und meistens auch nur dann, wenn die Affinität des Inhibitors zu dem zu inhibierenden Blutgerinnungsfaktor sehr hoch ist. Es hat sich gezeigt, dass die Inhibierung des Blutgerinnungsfaktors Xa zwar nicht der einzige Ansatzpunkt für eine effektive Antikoagulation von humanem Blut ex vivo ist, allerdings gegenüber der Inhibierung anderer Blutgerinnungsfaktoren der weitaus bevorzugteste. Obwohl diese Erfindung nicht durch den zugrunde liegenden Mechanismus eingeschränkt sein soll, könnten die oben beschriebenen Phänomene darauf zurückzuführen sein, dass sowohl der intrinsische als auch der extrinsische Weg der Blutgerinnung zum aktivierten Blutgerinnungsfaktor Xa führen. Somit ist die Aktivität des Blutgerinnungsfaktors Xa für die Bildung und Aktivierung von Thrombin unerlässlich, wodurch dessen Inhibierung zur Antikoagulation führt. Wird dagegen jedoch nicht der Blutgerinnungsfaktor Xa inhibiert, sondern zum Beispiel ein auf dem intrinsischen Weg aktiver Blutgerinnungsfaktor, dann kann zum Beispiel die Bildung und Aktivierung von Thrombin immer noch über die Aktivierung des extrinsischen Wegs erfolgen. Ein Grund für die besonders vorteilhafte Wirkung, die mit dem Inhibitor des Blutgerinnungsfaktors Xa gemäß Formel (I) erzielt wird, ist, dass dieser nicht nur eine starke Hemmwirkung auf den Blutgerinnungsfaktor Xa ausübt, sondern zusätzlich auch auf weitere Blutgerinnungsfaktoren, wie zum Beispiel Thrombin und Plasma-Kallikrein.

Dem Fachmann ist klar, dass die optimale Konzentration des einzusetzenden Inhibitors für das erfindungsgemäße Verfahren zur Antikoagulation von humanem Blut ex vivo variiert. Sie ist von dem Fachmann bekannten Faktoren abhängig, wie zum Beispiel der Hemmstärke und der Hemmspezifität des Inhibitors. Allerdings ist es kein Problem und erfordert auch keinen großen Aufwand, die optimale Konzentration des direkten Inhibitors des Blutgerinnungsfaktors Xa zur Antikoagulation von humanem Blut ex vivo herauszufinden. Dies kann zum Beispiel geschehen, indem man Blutproben mit unterschiedlichen Mengen des Inhibitors versetzt und so unterschiedliche Konzentrationen des Inhibitors in den Blutproben einstellt. Danach kann zu gewünschten Zeitpunkten einfach die Menge des Prothrombinfragments 1+2 (F 1+2) in den Proben mit Hilfe von zum Beispiel einem der kommerziell erhältlichen immunologischen Testkits bestimmt werden, um zu beurteilen, ob die Bildung von Thrombin inhibiert worden ist, also Antikoagulation erfindungsgemäß erfolgt ist. Auf diese Weise kann die optimale Konzentration des Inhibitors für das erfindungsgemäße Verfahren einfach und schnell ermittelt werden.

Für das Verfahren gemäß der vorliegenden Erfindung kann der erfindungsgemäße Inhibitor des Blutgerinnungsfaktors Xa zum Beispiel in einem geeigneten Solvens gelöst vorliegen. Vorzugsweise weist ein geeignetes Solvens eine physiologische Ionenstärke und/oder einen physiologischen pH-Wert auf. Beispiele hierfür sind physiologische Salzlösungen, wie zum Beispiel NaCl-Lösungen, oder Puffer. Ferner kann der Inhibitor des Blutgerinnungsfaktors Xa gemäß Formel (I) auch mit einem dem Fachmann bekannten Konservierungsmittel versetzt vorliegen.

Vorzugsweise wird die Antikoagulation nach dem erfindungsgemäßen Verfahren erreicht, indem 9 Teile Blut und 1 Teil einer den erfindungsgemäßen Inhibitor enthaltenden Lösung gemischt werden.

Aus praktischen Gründen ist es oft vorteilhaft, wenn der erfindungsgemäße Inhibitor direkt in einem Röhrchen zur Entnahme von Blutproben vorliegt. Das Röhrchen kann zum Beispiel eines der aus dem Stand der Technik bekannten und kommerziell erhältlichen Blutentnahmeröhrchen sein. Beispielhafte Blutentnahmeröhrchen umfassen Monovetten (Sarstedt AG & Co., Nümbrecht, Deutschland), Vacutainer-Systeme (z. B. der Firma Becton-Dickinson, Heidelberg, Deutschland) und modifizierte Bauweisen davon.

Vorzugsweise legt man den erfindungsgemäßen Inhibitor in einer solchen Menge oder einem solchen Volumen vor, dass die Konzentration des Inhibitors nach dem Befüllen des Röhrchens mit Blut ausreichend hoch ist, um die Koagulation des Blutes erfindungsgemäß zu verhindern.
Auf diese Weise wird es dem medizinischen Fachpersonal ermöglicht, eine Antikoagulation von humanem Blut ex vivo ohne aufwändige Arbeitsschritte zu erreichen.

In einer bevorzugten Ausführungsform wird das erfindungsgemäße Verfahren zur Antikoagulation von humanem Blut ex vivo zur Haltbarmachung und Lagerung von Blut verwendet. Bevorzugt handelt es sich bei dem haltbar zu machenden und zu lagernden Blut um Blutprodukte, wie Thrombozytenkonzentrate oder Thrombozytapheresekonzentrate.

Die jeweiligen Blutprodukte werden auf die für einen Fachmann bekannte Weise hergestellt.

Thrombozytenkonzentrate können zum Beispiel aus plättchenreichem Plasma (PRP) durch Zentrifugation bei hoher Umdrehungszahl, dem thrombozytenhaltigen Buffy-Coat (BC) von Blutkonserven oder aus Thrombozytapheresekonzentraten von einzelnen Spendern gewonnen werden.

Thrombozytapheresekonzentrate können zum Beispiel unter Verwendung eines handelsüblichen Zellseparators mittels Thrombozytapherese gewonnen werden.

Es ist zum Beispiel in diesen Fällen oft bevorzugt, wenn der erfindungsgemäße Inhibitor zur Antikoagulation von humanem Blut ex vivo in einer für das erfindungsgemäße Verfahren wirksamen Menge direkt in einem, aus dem Stand der Technik bekannten Blutbeutel vorliegt. Besonders bevorzugt liegt der erfindungsgemäße Inhibitor in einem Auffangbeutel zur Aufnahme von Blut vor. Insbesondere ist es bevorzugt, wenn dieser Auffangbeutel Teil eines dem Fachmann bekannten Aphereseschlauch-/beutelsystems ist. Dieses Aphereseschlauch-/beutelsystem kann sowohl einen als auch mehrere Auffangbeutel aufweisen, die den erfindungsgemäßen Inhibitor enthalten. Der erfindungsgemäße Inhibitor kann zum Beispiel sowohl in einem Beutel vorliegen, in dem das vom Spender entnommene Vollblut eingebracht wird, als auch in einem Beutel, in dem schließlich die Konzentrate aufgefangen werden.

In einer bevorzugten Ausführungsform liegt der erfindungsgemäße Inhibitor zur Antikoagulation von humanem Blut ex vivo in einem sterilen, ein Thrombozytenkonzentrat enthaltenden Auffangbeutel für die Infusion vor.

Gemäß einer weiteren Ausführungsform der Erfindung kann der erfindungsgemäße Inhibitor des Blutgerinnungsfaktors Xa zur Isolierung der zellulären Bestandteile des Blutes eingesetzt werden.

In einer weiteren bevorzugten Ausführungsform wird das erfindungsgemäße Verfahren zur Antikoagulation von humanem Blut ex vivo vorzugsweise für diagnostische Zwecke eingesetzt. Beispielsweise kann das Agens mit direkter Inhibitorwirkung auf den Blutgerinnungsfaktor Xa bzw. das mit diesem Agens erfindungsgemäß antikoaguliertes Blut zur Diagnostik von zellulären Blutbestandteilen eingesetzt werden.

Der Begriff "Diagnostik von zellulären Blutbestandteilen" oder Synonyme hiervon, beziehen sich erfindungsgemäß insbesondere auf Untersuchungen, die es dem medizinischen Fachmann erlauben, unmittelbar Aussagen über Art, Anzahl, Größe, Beschaffenheit und Funktionsfähigkeit (zum Beispiel Aktivität und Aktivierbarkeit) von zellulären Blutbestandteilen zu treffen.

Der Begriff zelluläre Blutbestandteile soll hierin zum Beispiel Thrombozyten, Erythrozyten, Retikulozyten und andere, dem Fachmann bekannte erythrozytäre Vorstufen, sowie Leukozyten, wie zum Beispiel Granulozyten, Lymphozyten, Monozyten, periphere Stammzellen und andere, dem Fachmann bekannte leukozytäre Vorstufen umfassen.

Die Verfahren zur Diagnose von zellulären Blutbestandteilen sind dem Fachmann gut bekannt. Beispielhaft genannt seien Verfahren zur Beurteilung der Thrombozytenfunktionen, die Zählung (zum Beispiel mikroskopische Zählung), morphologische Charakterisierung (zum Beispiel an gefärbten und ungefärbten Blutausstrichen) und Typisierung von Zellen im Blut, vorzugsweise mittels mikroskopischer, zytometrischer oder durchflusszytometrischer Verfahren, die elektronische Partikelzählung, Verfahren zur Erkennung einer in vivo Aktivierung der Hämostase und Fibrinolyse, Quantifizierung von Markern der aktivierten Thrombozyten, wie zum Beispiel Plättchenfaktor 4 (PF 4), β-Thromboglobulin, Serotonin, ADP/ATP, Prothrombin-Fragment F1+2, Thrombin-Antithrombin-Komplexe, D-Dimere, Fibrinogen-Spaltprodukte, lösliches Fibrin bzw. Fibrinmonomere, Plasmin-Plasmininhibitor-Komplexe, tPA-PAI-Komplexe, oder die elektronisch und zytochemisch (zum Beispiel Peroxidase) gestützte morphologische Charakterisierung und Zählung von beispielsweise periphären Leukozyten und anderen Zellen im Blut. Mittels Durchflusszytometrie lassen sich die zellulären Bestandteile beispielsweise diagnostizieren, indem Bestandteile von Zelloberflächen mit markierten Antikörpern (zum Beispiel fluoreszenzmarkierten monoklonalen Antikörpern) charakterisiert und die derart markierten Zellen im Durchflusszytometer gezählt (zum Beispiel durch elektronische Zählung) werden.

Das gemäß dem Verfahren der vorliegenden Erfindung antikoagulierte Blut kann beispielsweise für alle derzeit gebräuchlichen Verfahren zur Beurteilung der Thrombozytenfunktionen eingesetzt werden. Beispielhafte Verfahren umfassen die spontane Thrombozytenaggregation nach Breddin, die induzierte Thrombozytenaggregation nach Born, die Impedanz-Vollblutaggregometrie, die Sekretion (Freisetzung) von thrombozytären Inhaltsstoffen (zum Beispiel ADP/ATP, Plättchenfaktor 4, β-Thromboglobulin, Serotonin), die Thrombozytenausbreitung (zum Beispiel nach Breddin), die Thrombozytenadhäsion, die Bestimmung von Thrombozytenoberflächen-Proteinen nach Aktivierung (zum Beispiel durch Durchflusszytometrie), die Messung der "In vitro-Blutungszeit" zum Beispiel mit dem Platelet Function Analyzer 100 (PFA-100; Dade Behring), die Thrombelastographie, die Gerinnsel-Retraktion, den Platelet Reactivity Index (zum Beispiel nach Grotemeyer, Wu & Hoak) und den Platelet Adhesion Assay (zum Beispiel nach Nowak et al. Semin. Thromb. Hemost. 31, 470, 2005).

Zur Messung bevorzugt eingesetzte Instrumente umfassen den Clot Signature Analyser (CSA), den Cone and Platelet Analyser (IMPACT), Durchflusszytometer, Ichor-Platelet Works, Laser Platelet Aggregometer (PA-200), Hemostasis Analysis System, Hemostasus, Ultegra (RPFA), Thrombotic Status Analyser (TSA), Hemodyne Platelet Analysis System, VerifyNow, Platelet-Stat, Multiplate analyzer und andere Geräte für die Impendanz-Aggregometrie, sowie diverse Geräte zur turbidimetrischen Bestimmung der Thrombozytenaggregation nach Born.

Die Erfindung umfasst daher auch ein Verfahren zur Diagnostik von zellulären Blutbestandteilen, bei dem man entnommenes Blut ex vivo antikoaguliert, indem man dem Blut wenigstens ein Agens mit direkter Inhibitorwirkung auf den Blutgerinnungsfaktor Xa gemäß Formel (I) zusetzt, und anschließend die zellulären Bestandteile des Bluts untersucht. In einer bevorzugten Ausführungsform wird das Verfahren zur Diagnostik von Thrombozyten eingesetzt, wobei mit dem erfindungsgemäß antikoagulierten Blut zum Beispiel die vorstehend beschriebenen Thrombozytenfunktionstests durchgeführt werden.

Eine weitere bevorzugte Ausführungsform der Erfindung ist ein Kit, der einen erfindungsgemäßen Inhibitor des Blutgerinnungsfaktors Xa gemäß Formel (I) und ein Mittel zur Untersuchung von zellulären Blutbestandteilen umfasst. Das in dem Kit enthaltene Mittel zur Untersuchung von zellulären Blutbestandteilen umfasst insbesondere eine zur Diagnostik von zellulären Blutbestandteilen geeignete und gegebenenfalls an ein Gerät zur Diagnostik von zellulären Blutbestandteilen angepasste Einheit (zum Beispiel eine Messzelle oder Kartusche). In einer bevorzugten Ausführungsform enthält der Kit als Mittel zur Untersuchung von zellulären Blutbestandteilen ein Mittel zur Thrombozytenfunktionsdiagnostik. Dieses Mittel zur Thrombozytenfunktionsdiagnostik kann bspw. eine Messzelle, eine Kartusche oder eine andere Einheit sein, die an herkömmliche Geräte zur Messung der Thrombozytenfunktion angepasst ist und für die Beurteilung der Thrombozytenfunktion eingesetzt werden kann. In einer bevorzugten Ausführungsform umfasst der Kit als Mittel zur Thrombozytenfunktionsdiagnostik oder als weiteren Bestandteil des Kits einen Aktivator der Thrombozyten. Dieser Aktivator kann in fester Form, gelöst oder auf einem als Mittel zur Thrombozytenfunktionsdiagnostik fungierenden Träger vorliegen. Der Träger kann zum Beispiel eine dem Fachmann bekannte Messzelle zur Messung von Verschlusszeiten oder eine weitere, für die Messung der Thrombozytenaktivität geeignete, gegebenenfalls an eine aus dem Stand der Technik bekannte Messvorrichtung angepasste Einheit sein. Der Aktivator selbst kann ein dem Fachmann bekannter Aktivator der Thrombozyten sein. Beispielhaft seien ADP, Arachidonsäure (AA), Thrombospondin, TRAP (Thrombin Receptor Activating Protein), Collagen oder Epinephrin genannt.

All die oben aufgeführten Tests zur Messung der Thrombozytenfunktionen werden derzeit in plättchenreichem Citratplasma oder in Citrat-Vollblut durchgeführt. Das erfindungsgemäße Verfahren zur Antikoagulation von humanem Blut ex vivo bietet gegenüber dem Stand der Technik den Vorteil, dass zwar komplett die plasmatische Gerinnung (also die Thrombinbildung und in der Folge die Fibrinbildung durch Thrombin) gehemmt wird, nicht aber die Aktivierbarkeit der Thrombozyten durch geeignete Induktoren wie zum Beispiel ADP, Collagen, Arachidonsäure, Thrombospondin, Epinephrin oder TRAP (Thrombin Receptor Activating Protein). Im Gegensatz dazu entstehen im Citratmilieu immer mit der Zeit Spuren von Thrombin, das ebenfalls ein starker Aktivator der Thrombozyten ist bzw. verlieren die Thrombozyten ihre Funktionsfähigkeit durch Calcium-Entzug. Das erfindungsgemäße Verfahren zur Antikoagulation von humanem Blut ex vivo hat daher den wesentlichen Vorteil, dass der erfindungsgemäße Inhibitor vor der Durchführung von Tests zur Diagnose von zellulären Blutbestandteilen, wie zum Beispiel Thrombozytenfunktionstests, weder entfernt noch inaktiviert werden muss, da er die Thrombozytenfunktion selbst nicht beeinflusst.

Die oben genannten Analyseverfahren werden grundsätzlich wesentlich gestört, wenn bei oder nach der Blutabnahme ex vivo bzw. in vitro eine zusätzliche Aktivierung der Thrombozyten erfolgt. Dadurch werden fälschlicherweise zu hohe Werte gemessen, die eine falsche Laborbefundinterpretation zur Folge haben, nämlich eine vermeintlich starke Aktivierung der Hämostase (Blutstillung) in vivo. Durch das erfindungsgemäße Verfahren zur Antikoagulation von humanem Blut ex vivo wird die Aggregabilität der Thrombozyten vollständig erhalten und die Bildung von Thrombin und Fibrin und damit die Aktivierung der Thrombozyten derart verhindert, dass die mit dem erfindungsgemäß antikoagulierten Blut gemessenen Werte am ehesten die Verhältnisse in vivo widerspiegeln.

### Beispiele:

Die vorliegende Erfindung wird im Folgenden anhand von Beispielen illustriert, wobei diese jedoch nicht als einschränkend verstanden werden sollen.

In den Beispielen 1-5 wurden als Inhibitoren kommerziell erhältliche Inhibitoren des Blutgerinnungsfaktors Xa eingesetzt.

In den Beispielen 6-11 wurde als Inhibitor ein Inhibitor des Blutgerinnungsfaktors Xa gemäß Formel (I) verwendet. Der Inhibitor des Blutgerinnungsfaktors Xa gemäß Formel (I) inhibiert neben dem Blutgerinnungsfaktor Xa weitere, gleiche Blutgerinnungsfaktoren.

Figur 1 zeigt den Einfluss von Pefabloc tPA/Xa, einem Inhibitor des Blutgerinnungsfaktors Xa, auf die Bildung von Thrombin nach Aktivierung mit Kaolin in mit Hirudin antikoaguliertem Plasma. In der Legende bedeuten Kontrolle - A: Hirudin-antikoaguliertes Plasma ohne Aktivierung durch Kaolin-Zugabe, Kontrolle + A: Hirudin-antikoaguliertes Plasma plus Kaolin, und die Zahlenwerte: Mit Kaolin versetztes, Hirudin-antikoaguliertes Plasma plus Pefabloc tPA/Xa in den angegebenen Konzentrationen.

Figur 2 zeigt den Einfluss von CJ-PK (Benzylsulfonyl-D-seryl-carbobenzoxylysyl-4-amidinobenzylamid, Haemochrom GmbH, Essen), einem Inhibitor von Plasma-Kallikrein, auf die Bildung von Thrombin nach Aktivierung mit Kaolin in mit Hirudin antikoaguliertem Plasma. In der Legende bedeuten Kontrolle - A: Hirudin-antikoaguliertes Plasma ohne Aktivierung durch Kaolin-Zugabe, Kontrolle + A: Hirudin-antikoaguliertes Plasma plus Kaolin, und die Zahlenwerte: Mit Kaolin versetztes, Hirudin-antikoaguliertes Plasma plus CJ-PK in den angegebenen Konzentrationen.

Figur 3 zeigt den Einfluss von Otamixaban, einem Inhibitor des Blutgerinnungsfaktors Xa, auf die Bildung von Thrombin nach Aktivierung mit Kaolin in mit Hirudin antikoaguliertem Plasma. In der Legende bedeuten Kontrolle - A: Hirudin-antikoaguliertes Plasma ohne Aktivierung durch Kaolin-Zugabe, Kontrolle + A: Mit Kaolin versetztes, Hirudin-antikoaguliertes Plasma, und die Zahlenwerte: Hirudin-antikoaguliertes Plasma plus Kaolin und Otamixaban in den angegebenen Konzentrationen.

Figur 4 zeigt die Bildung des Prothrombinfragments F1+2 (F1+2) nach der Antikoagulation von humanem Venenblut mit Otamixaban (Konzentration im antikoagulierten Blut = 500 µM, 50 µM und 5,0 µM).

Figur 5 zeigt die Bildung des Prothrombinfragments F1+2 (F1+2) nach der Antikoagulation von humanem Venenblut mit CJ-FXa (Konzentration im antikoagulierten Blut = 500 µM und 50 µM).

Figur 6 zeigt den Einfluss von BAPA' auf die Bildung von Thrombin nach Aktivierung mit Kaolin in mit Hirudin antikoaguliertem Plasma. In der Legende bedeuten Kontrolle - A: Hirudin-antikoaguliertes Plasma ohne Aktivierung durch Kaolin-Zugabe, Kontrolle + A: Hirudin-antikoaguliertes Plasma plus Kaolin, und die Zahlenwerte: Mit Kaolin versetztes, Hirudin-antikoaguliertes Plasma plus BAPA' in den angegebenen Konzentrationen.

Figur 7 zeigt die Bildung des Prothrombinfragments F1+2 (F1+2) nach der Antikoagulation von humanem Venenblut mit BAPA' (Konzentration im antikoagulierten Blut = 500 µM, 50 µM und 5,0 µM).

Figur 8 zeigt die von verschiedenen Agonisten induzierte Thrombozytenaggregation in mit BAPA' bzw. Zitrat antikoaguliertem Blut. In der Legende bedeuten TPA:
Turbidimetrische Plättchenaggregation, IPA: Impedanz-Vollblut-Plättchenaggregation und AA: Arachidonsäure.

Figur 9 zeigt den Anteil an aggregierten Thrombozyten (Plättchen) in mit BAPA' bzw. Zitrat antikoaguliertem Blut, wobei die Thrombozytenaggregation mit verschiedenen Agonisten induziert wurde. In der Legende bedeuten PPA: Partikelzahl-Plättchenaggregation und AA: Arachidonsäure.

Figur 10 zeigt die Verschlusszeiten von mit BAPA' bzw. Zitrat anikoaguliertem Blut für mit verschiedenen Agonisten beschichteten Membranen. In der Legende bedeuten CADP: Collagen-ADP, CEPI: Collagen-Epinephrin, CT: Closure Time (Verschlusszeit), und PFA-100: Platelet Function Analyzer 100.

### Beispiel 1: Hemmwirkung von beispielhaft eingesetzten Inhibitoren gegenüber den humanen Gerinnungsfaktoren Xa, VIIa, XIa, XIIa und Plasma-Kallikrein (PK)

Bei den hierbei verwendeten Inhibitoren handelt es sich um die kommerziell erhältlichen, beispielhaften Inhibitoren Pefabloc tPA/Xa (2,7-Bis(4-amidinobenzyliden)-cycloheptanon-(1), Pentapharm Ltd. Basel, Schweiz), CJ-FXa (Benzylsulfonyl-D-arginyl-glycyl-4-amidinobenzylamid, Haemochrom GmbH, Essen, Deutschland) und CJ-PK (Benzylsulfonyl-D-seryl-carbobenzoxylysyl-4-amidinobenzylamid, Haemochrom GmbH, Essen, Deutschland). Von DX-9065a ((+)-(2S)-2-[4-[[(3S)-1-Acetimidoyl-3-pyrrodinyl]oxy]phenyl]-3-[7-amidino-2-naphthyl]propansäure, Daiichi Pharmaceutical Company Ltd. Tokio, Japan), und Otamixaban (2-(R)-(3-carbamimidoylbenzyl)-3-(R)-[4-(1-oxypyridin-4-yl)benzoylamino]-buttersäure-methylester, Sanofi-Aventis GmbH, Frankfurt, Deutschland), die sich beide in klinischen Prüfungen befinden, standen Forschungsproben zur Verfügung. Um die Hemmwirkung dieser beispielhaft eingesetzten Inhibitoren auf die Blutgerinnungsfaktoren Plasma-Kallikrein, VIIa, Xa, XIa und XIIa zu untersuchen, wurden die Kᵢ-Werte der jeweiligen Enzym-Inhibitor-Komplexe ermittelt. Die Bestimmung der Kᵢ-Werte wurde bei 25 °C in Tris-Puffer (0,05 mol/l, pH 8,0; enthält 0,154 mol/l NaCl und 5 % Ethanol) durchgeführt. Die Enzyme stammten von der Firma Haemochrom Diagnostica GmbH (Essen, D) und waren in 0,154 mol/l NaCl gelöst. Die Substrate (Pentapharm AG, Basel, CH) waren in H₂O, der Inhibitor im obengenannten Tris-Puffer gelöst. Für die einzelnen Enzyme wurden die folgenden Substrate benutzt:

| | Substrat | |
|---|---|---|
| **Enzym** | Chemische Bezeichnung | Handelsname |
| Plasma-Kallikrein | Bz-Pro-Phe-Arg-pNA | Chromozym PK |
| Faktor VIIa | CH₃SO₂-D-Phe-Gly-Arg-pNA | Chromozym tPA |
| Faktor Xa | CH₃OCO-D-Cha-Gly-Arg-pNA | Pefachrome FXa |
| Faktor XIa | H-D-Lys(Cbo)-Pro-Arg-pNA | Chromozym PCa |
| Faktor XIIa | H-D-HHT-Gly-Arg-pNA | Chromozym XII |

| | | |
|---|---|---|
| Bz = Benzyl-, Lys(Cbo) = Omega-Carbobenzoxylysin, HHT, Hexahydrotyrosin, Cha, Cyclohexylalanin, pNA = para-Nitroanilin | | |

Für die Bestimmung wurden 200 µl Inhibitor-Lösung mit 25 µl Substrat gemischt und die Reaktion durch Zugabe von 50 µl Enzym gestartet. Nach einer Reaktionszeit von 3 - 5 min wurden 25 µl Essigsäure (50 %) zugegeben und die Extinktion bei 405 nm mit einem microplate reader (iEMS Reader MF 1401, Labsystems, Helsinki, Finnland) gemessen. Die Auswertung erfolgte graphisch entsprechend Dixon (Biochem. J., 55, 170, 1953).

Die für die beispielhaft eingesetzten Inhibitoren bestimmten Kᵢ-Werte sind in Tabelle 1 dargestellt.

**Tabelle 1**

| | Kᵢ (µM) | | | | |
|---|---|---|---|---|---|
| Inhibitor | Faktor Xa | Faktor VIIa | Faktor XIa | Faktor XIIa | PK |
| DX-9065a | 0,007 | 92 | 2,3 | 0,34 | 0,53 |
| Pefabloc tPA/Xa | 0,0051 | 140 | 42 | 0,36 | 0,72 |
| CJ-FXa | 0,0035 | 9,6 | 0,80 | 0,35 | 0,084 |
| CJ-PK | 2,9 | 3,8 | 0,25 | 12 | 0,0035 |
| Otamixaban | 0,00050 | 19 | 23 | > 1000 | 0,026 |

| | | | | | |
|---|---|---|---|---|---|
| PK = Plasma-Kallikrein | | | | | |

### Beispiel 2: Hemmung der Thrombin-Bildung in Hirudin-antikoaguliertem Plasma nach Aktivierung mittels Kaolin durch Inhibitoren von Blutgerinnungsfaktoren

In diesem Beispiel wurde die Thrombin-Bildung in mit Hirudin antikoaguliertem Plasma durch Zugabe von Kaolin aktiviert und die Fähigkeit der Inhibitoren, die Thrombin-Bildung zu inhibieren, untersucht.

Zunächst wurde von einem Patienten entnommenes Blut mit Hilfe des Thrombin-Inhibitors Hirudin antikoaguliert. Dazu wurden 9 Teile Blut mit 1 Teil Hirudin-Lösung (rekombinantes Hirudin HBW 023, Hoechst, Frankfurt, 2000 E/ml physiologischer NaCl-Lösung) gemischt und danach 10 Minuten bei 1300 Umdrehungen pro Minute (U/min) zentrifugiert. 900 µl des sich im Überstand befindlichen, mit Hirudin antikoagulierten Plasmas wurden in Polypropylen-Röhrchen mit 50 µl Inhibitorlösung bzw. 0,9 %iger NaCl-Lösung sowie 50 µl Kaolin-Suspension (aus APTT-Test von Roche Diagnostics, vor Gebrauch 1:250 verdünnt mit physiologischer NaCl-Lösung) versetzt und bei 37°C unter leichtem Schütteln inkubiert. Kaolin fungiert dabei als unphysiologische Oberfläche, die die Blutgerinnung aktiviert, wodurch die Thrombin-Bildung schließlich stimuliert wird. Zu bestimmten Zeitpunkten wurde die Menge des gebildeten Thrombins anhand der Bildung des Prothrombinfragments F1+2 immunologisch bestimmt (Enzygnost F1+2 micro, Dade Behring GmbH, Marburg). Diese Bestimmung basiert auf einem Enzymimmunoassay nach dem Sandwich-Prinzip. Dazu wurden zu bestimmten Zeitpunkten aliquote Volumina (600 µl) mit 0,1 M EDTA (40 µl) antikoaguliert und bei - 20 °C aufbewahrt. Zur Bestimmung der Konzentration des Prothrombinfragments F1+2 wurden 50 µl Plasma (gegebenenfalls verdünnt mit PBS, phosphate-buffered saline) mit monoklonalen Antikörpern gegen das humane Prothrombinfragment F1+2, die auf einer im Testkit enthaltenen Mikrotiterplatte fixiert waren, inkubiert. Nach einem Waschschritt mit PBS, der zur Entfernung von nicht von den Antikörpern gebundenem Probenmaterial diente, wurde mit Peroxidasekonjugierten Antikörpern gegen humanes Prothrombin, die an die freien F1+2-Determinanten binden, inkubiert. Nach einem weiteren Waschvorgang, bei dem die ungebundenen Enzym-konjugierten Antikörper entfernt werden, wurde Chromogen (o-Phenylendiamin/H₂O₂-Lösung) zugesetzt, um mit der an die Antikörper gegen humanes Prothrombin gebundene Peroxidase eine Farbreaktion zu erzeugen, deren Intensität proportional zur Konzentration des Prothrombinfragments F1+2 in der Probe ist. Diese wurde im Anschluss photometrisch bei einer Wellenlänge von 492 nm bestimmt.

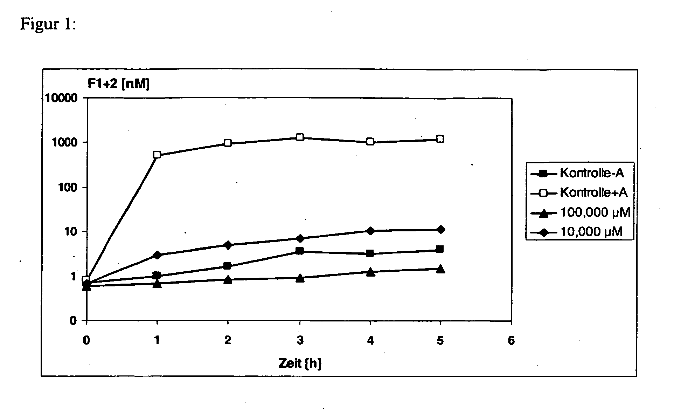

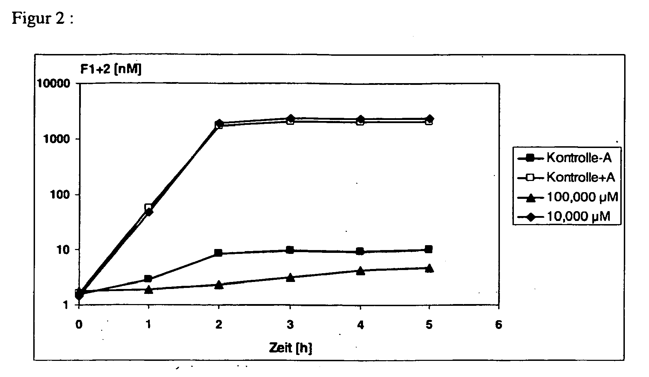

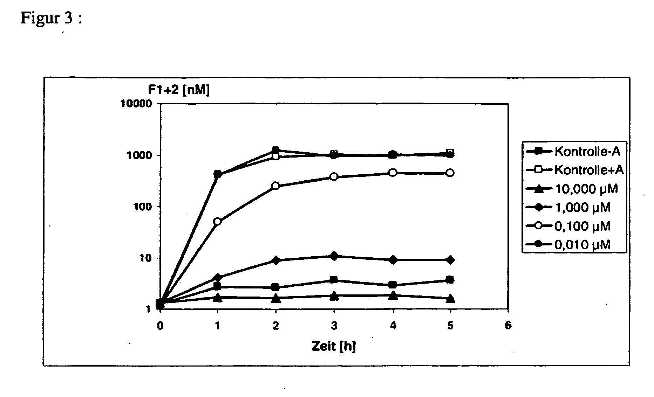

Wie aus den Figuren 1 bis 3 ersichtlich ist, sind die beispielhaften Inhibitoren in der Lage, die durch die Aktivierung mit Kaolin stimulierte Thrombin-Bildung effektiv zu unterdrücken. So sind Hemmstoffe des Blutgerinnungsfaktors Xa mit Kᵢ-Werten bis 10 nM, wie der in Tabelle 1 beispielhaft gezeigte Inhibitor Pefabloc tPA/Xa (Fig. 1) in der Lage bei einer Konzentration von 100 µM die Thrombin-Bildung total zu unterdrücken. Auch sehr starke Hemmstoffe der Enzyme der Frühphase der Gerinnung wie der Plasma-Kallikrein-Hemmstoff CJ-PK (Tabelle 1) unterdrücken bei einer Konzentration von 100 µM die Thrombin-Bildung effektiv (Fig. 2). Als besonders geeignet zur Blockade der Thrombin-Bildung nach Kaolin-Aktivierung erweisen sich Substanzen, die wie Otamixaban den Blutgerinnungsfaktor Xa mit einem Kᵢ-Wert < 0,1 nM hemmen (Fig. 3). Bei der Interpretation der in diesem Beispiel erhaltenen Ergebnisse muss berücksichtigt werden, dass die Versuche in diesem Beispiel nicht in physiologischem Milieu durchgeführt wurden, sondern unter Verwendung von Kaolin, dass ein unphysiologischer, starker Aktivator der Thrombin-Bildung ist. Diese Ergebnisse zeigen daher eindrucksvoll, dass Blutplasma trotz der starken unphysiologischen Aktivierung der Thrombin-Bildung durch Kaolin mit Hilfe des erfindungsgemäßen Verfahrens erfolgreich antikoaguliert werden kann.

### Beispiel 3: Lagerung von humanem Venenblut nach Antikoagulation mit verschiedenen Inhibitoren

In diesem Experiment wurde untersucht, wie lange sich humanes Venenblut lagern lässt, ohne dass es dabei zur Bildung von Thrombin oder zur Aktivierung der Thrombozyten kommt.

Dazu wurde von einem Patienten entnommenes Blut (9 Teile) mit Inhibitor-Lösung (0,05 mM, 0,5 mM bzw. 5 mM in physiologischer NaCl-Lösung; 1 Teil) gemischt. Ein Teil des Blutes wurde bei 25 °C, der andere im Kühlschrank bei 4 °C aufbewahrt. Die Menge des gebildeten Thrombins wurde im Verlauf der Lagerung anhand der Bildung des Prothrombinfragments F1+2 immunologisch ermittelt (Enzygnost F1+2 micro, Dade Behring GmbH, Marburg). Es konnte gezeigt werden, dass die beispielhaft eingesetzten Hemmstoffe CJ-FXa und Otamixaban in der Lage sind, antikoaguliertes Blut bei einer Konzentration im Blut von 500 µM über mehr als 48 Stunden ungeronnen zu halten (Tabelle 2). Dabei spielte es keine Rolle, ob die Blutproben bei Zimmertemperatur (ca. 25 °C) oder bei 4 °C aufbewahrt wurden. Bei einer Konzentration von 50 µM verhindert Otamixaban die Gerinnung für mehr als 24 Stunden, CJ-FXa nur für etwa 24 Stunden.

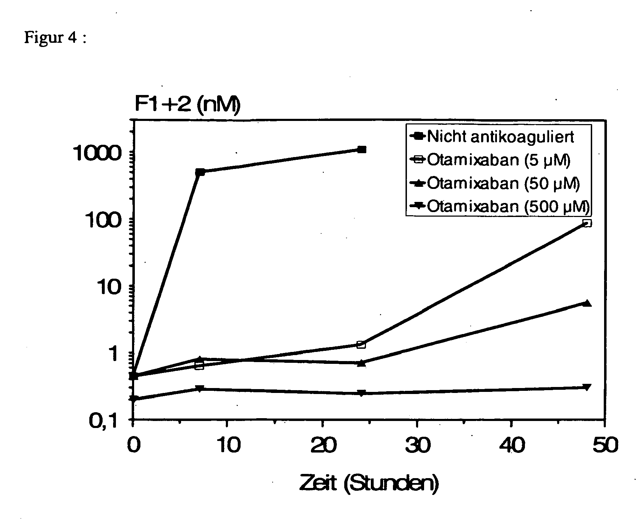

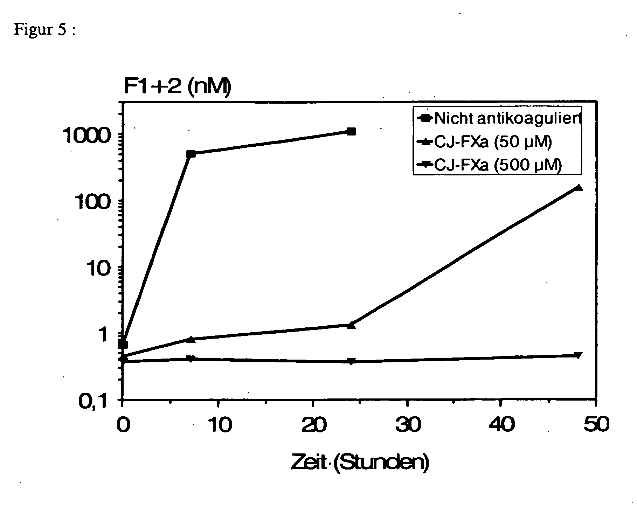

Wie beispielhaft für Otamixaban (Fig. 4) und CJ-FXa (Fig. 5) gezeigt wurde, wird in dem jeweiligen Zeitraum weniger als 1 nM Thrombin (Nachweis über F1+2-Fragment) neu gebildet. Die anderen beispielhaft geprüften Hemmstoffe DX-9065a und Pefabloc tPA/Xa sind nicht in der Lage, in den angegebenen Konzentrationen die Gerinnung für mehr als 12 Stunden zu verhindern.

**Tabelle 2**

| | Konzentration | Dauer der Antikoagulation (h) | |
|---|---|---|---|
| Inhibitor | im Blut (µM) | 4°C | 25°C |
| DX 9065a | 500 | n.b. | 7- 12 |
| DX 9065a | 50 | n.b. | 5-7 |
| Pefabloc tPA/Xa | 500 | n.b. | 7-10 |
| CJ-FXa | 500 | > 48 | > 48 |
| CJ-FXa | 50 | n.b. | 12-24 |
| CJ-FXa + CJ-PK | jeweils 50 | n.b | 24-32 |
| Otamixaban | 500 | > 48 | > 48 |
| Otamixaban | 50 | n.b. | 24-32 |
| Otamixaban | 5 | n.b. | 12-24 |

Diese Ergebnisse zeigen auch, dass die Effektivität des erfindungsgemäßen Verfahrens zur Antikoagulation von humanem Blut ex vivo mit dem Kᵢ-Wert des Inhibitors gegenüber dem Blutgerinnungsfaktor Xa positiv korreliert. Es soll an dieser Stelle außerdem angemerkt werden, dass sich das erfindungsgemäße Verfahren mit jedem direkten Inhibitor des Blutgerinnungsfaktors Xa durchführen lässt. So lässt sich die Antikoagulation von humanem Blut ex vivo auch mit direkten Inhibitoren des Blutgerinnungsfaktors Xa erreichen, die Kᵢ-Werte über 10 nM aufweisen. In diesen Fällen muss lediglich die Konzentration dieses Inhibitors im Blut entsprechend erhöht werden. Daher ist es zum Beispiel aus Kostengründen oft vorteilhaft, wenn stärkere direkte Inhibitoren des Blutgerinnungsfaktors Xa (Kᵢ-Wert < 10 nM) in geringeren Konzentrationen eingesetzt werden.

### Beispiel 4: Induzierte Aktivierung der Thrombozyten nach der Antikoagulation mit direkten Inhibitoren des Blutgerinnungsfaktors Xa

In diesem Experiment wird gezeigt, dass sich die Thrombozyten in dem erfindungsgemäß antikoagulierten Blut durch Induktion problemlos aktivieren lassen, während dies bei mit Citrat antikoaguliertem Blut nicht über einen längeren Zeitraum der Fall ist.

Dazu wurden von 12 gesunden Probanden innerhalb 1 Stunde Blut entnommen. Es wurde jeweils 1 Aliquot der 12 Blutproben mit Citrat, 1 anderes Aliquot der 12 Blutproben mit einem direkten Inhibitor des Blutgerinnungsfaktors Xa antikoaguliert. Dazu wurden 9 Teile Blut mit 1 Teil Citrat (0,106 mol/l) bzw. 1 Teil Inhibitor-Lösung in der geeigneten Konzentration vermischt. Plättchenreiches Plasma (PRP) wurde durch Zentrifugation bei 164 x g über 10 Minuten hergestellt. Die Aktivierung der Thrombozyten in PRP erfolgte durch induzierte Aggregation nach Born mit 5 µmol/l ADP, 2 µg/ml Collagen und 0,5 mmol/l Arachidonsäure. Impedanz-Vollblutaggregometrie wurde mit dem Multiplate Aggregometer und mit 6,5 µmol/l ADP, 3,2 µg/ml Collagen und 0,5 mmol/l Arachidonsäure durchgeführt. Die Analysen erfolgten 1, 2, 3, 4, 5, 10 und 24 Stunden nach der Blutentnahme. Während die induzierten Aggregationen in dem mit Citrat antikoagulierten Blut 24 Stunden nach der Blutabnahme auf 20 bis 30% des Ausgangswertes abgefallen waren, änderte sich das Ausmaß der Aggregation in dem erfindungsgemäß antikoagulierten Blut nicht.

24 Stunden nach der Blutabnahme zeigten wie 1 Stunde nach der Blutabnahme 70 % der Thrombozyten in dem erfindungsgemäß antikoagulierten RPR eine normale Ausbreitung auf Kunststoff-Objektträgern. Demgegenüber waren 24 Stunden nach der Blutabnahme weniger als 10 % der Thrombozyten in mit Citrat antikoaguliertem RPR noch ausgebreitet.

### Beispiel 5: Haltbarmachung und Lagerung von Thrombozytapheresekonzentraten

In diesem Experiment wird gezeigt, dass sich Blutprodukte, wie zum Beispiel Thrombozytapheresekonzentrate unter Verwendung des erfindungsgemäßen Verfahrens zur Antikoagulation von humanem Blut ex vivo über einen langen Zeitraum haltbar machen und lagern lässt.

Ein Thrombozytapheresekonzentrat, das aus in Plasma suspendierten Thrombozyten besteht, wurde durch Thrombozytapherese mittels eines handelsüblichen Zellseparators unter Verwendung von zunächst Citrat als Antikoagulans hergestellt. In einem geschlossenen Thrombozytapherese-Beutelsystem wurde ein Satellitenbeutel integriert, der Calciumchlorid und einen erfindungsgemäßen direkten Inhibitor des Blugerinnungsfaktors Xa in physiologischer Lösung enthielt. Unmittelbar nach Beendigung der Thrombozytapherese wurde die Lösung im Satellitenbeutel dem Thrombozytapheresekonzentrat zugesetzt. Die Zugabe von Calciumionen (Recalcifizierung) stellte eine physiologische Calciumionenkonzentration im Thrombozytapheresekonzentrat ein, die zur Gewährleistung einer optimale Thrombozytenfunktion diente. Die Antikoagulation des nun wieder gerinnbaren Plasmas, in dem die Thrombozyten suspendiert waren, wurde erfindungsgemäß durch die Zugabe des direkten Inhibitors des Blutgerinnungsfaktors Xa verhindert.

Das Thrombozytapheresekonzentrat wurde unter bei 22 ± 2°C unter ständiger Agitation gelagert. Nach 10-tägiger Lagerung besaß das Thrombozytapheresekonzentrat, das erfindungsgemäß mit einem direkten Inhibitor des Blutgerinnungsfaktors Xa antikoaguliert wurde, noch die gleiche Wirksamkeit wie konventionelle Thrombozytapheresekonzentrate nach 5 Tagen.

So zeigten die erfindungsgemäß antikoagulierten Thrombozytapheresekonzentrate auch nach 10-tägiger Lagerung noch das "Swirling-Phänomen" (das "Swirling-Phänomen" basiert auf der Lichtstreuung von bewegten, eine normale Morphologie aufweisenden Thrombozyten, und verschwindet, wenn die normalerweise scheibenförmigen Thrombozyten eine abnormale, kugelförmige Gestalt annehmen) und wiesen einen pH-Wert > 6,7 auf. Die Thrombozytenzahl war dabei immer größer als > 2 x 10¹¹/Einheit. Die Aggregation der Thrombozyten war durch Induktion mit 2 µg/ml Collagen auslösbar, wobei die Maximalamplitude 25 % betrug. Ferner konnte die Thrombozytenaggregation auch mit 5 bzw. 10 µmol/l ADP induziert werden.

Diese Ergebnisse zeigen, dass sich Blutprodukte durch das erfindungsgemäße Verfahren über einen langen Zeitraum haltbar machen und lagern lassen, ohne dass es dabei zur Antikoagulation kommt, und ohne dass die Aktivierbarkeit der Thrombozyten dadurch beeinträchtigt wird.

### Beispiel 6: Synthese des erfindungsgemäßen Inhibitors BAPA'

Der für die nachfolgenden Untersuchungen exemplarisch eingesetzte Inhibitor gemäß Formel (II), BAPA', wurde durch Kopplung von Benzylsulfonyl-D-Arg(Pbf)-OH (hergestellt entsprechend Schweinitz et al., Med. Chem. 2006, 2, 349-361) und H-Pro-4-Amidinobenzylamid × 2 HCl (hergestellt entsprechend WO 02/059065) synthetisiert, wobei Pbf eine 2, 2', 4, 6, 7-Pentamethyldihydro-benzofuran-5-sulfonyl-Schutzgruppe ist. Dazu wurden 0,5 g (0,861 mmol) Benzylsulfonyl-D-Arg(Pbf)-OH und 289 mg (0,904 mmol) H-Pro-4-Amidinobenzylamid x 2 HCl in 15 ml DMF gelöst. Danach wurden bei 0 °C 326 mg (0,861 mmol) 2-(1H-Benzotriazol-1-yl)1,1,3,3-Tetramethyluronium Hexafluorophosphat und 300 µl (1,722 mmol) Diisopropylethylamin dazugegeben. Die Mischung wurde 30 min bei 0 °C und weitere 5 h bei Zimmertemperatur (22 ± 2°C) gerührt. Mit Vakuum wurde das Lösungsmittel entfernt und der ölige Rückstand ohne weitere Reinigung mit 10 ml Trifluoressigsäure versetzt. Nach 1,5 h Rühren bei Raumtemperatur (22 ± 2 °C) wurde das Lösungsmittel im Vakuum eingeengt und der Rückstand anschließend mit Ether versetzt. Das ausfallende Rohprodukt wurde mit präparativer RP-HPLC getrennt. Die Fraktionen, die BAPA' enthielten, wurden vereint und nach teilweiser Entfernung des Lösungsmittels lyophilisiert.

Zur präparativen RP-HPLC wurde eine HPLC-Anlage der Firma Waters und eine Säule Saphir 110 (10µm C18) 50 x 300 mm der Firma Grom verwendet. Die Detektion erfolgte bei 220 nm. Als Elutionsmittel wurde Wasser mit 0,1 % TFA (A) und Acetonitril mit 0,1 % TFA (B) bei einer Flussrate von 10 oder 20 ml/min und einem geeigneten Gradienten eingesetzt. Die Massenspektren wurden auf einem ZQ 4000 der Firma Waters gemessen. Die Synthese lieferte eine Ausbeute an BAPA' (Molekulargewicht als 2 × TFA-Salz: 784,7 g/mol) von 295 mg (entsprechend 0,376 mmol).

Die berechnete Molmasse beträgt MS = 556,26. Im Massenspektrum wurde eine Molmasse von MS = 557,3 (M+H)⁺ gefunden.

### Beispiel 7: Hemmwirkung von BAPA' gegenüber den humanen Gerinnungsfaktoren Xa, VIIa, XIa, XIIa, Plasma-Kallikrein (PK) und Thrombin

Um die Hemmwirkung des beispielhaft eingesetzten Inhibitors BAPA' auf die Blutgerinnungsfaktoren Plasma-Kallikrein, VIIa, Xa, XIa, XIIa und Thrombin zu untersuchen, wurden die Kᵢ-Werte der jeweiligen Enzym-Inhibitor-Komplexe ermittelt.

Die Bestimmung der Kᵢ-Werte wurde bei 25 °C in Tris-Puffer (0,05 mol/l, pH 8,0; enthält 0,154 mol/l NaCl und 5 % Ethanol) durchgeführt. Die Enzyme stammten von der Firma Haemochrom Diagnostica GmbH (Essen, D), nur Thrombin wurde im eigenen Labor nach Walsmann (Pharmazie 23, 401-402, 1968) hergestellt. Alle Enzyme waren in 0,154 mol/l NaCl gelöst. Die Substrate (Pentapharm AG, Basel, CH) waren in H₂O, der Inhibitor im oben genannten Tris-Puffer gelöst. Für die einzelnen Enzyme wurden die folgenden Substrate benutzt:

| | Substrat | |
|---|---|---|
| **Enzym** | Chemische Bezeichnung | Handelsname |
| Plasma-Kallikrein | Bz-Pro-Phe-Arg-pNA | Chromozym PK |
| Faktor VIIa | CH₃SO₂-D-Phe-Gly-Arg-pNA | Chromozym tPA |
| Faktor Xa | CH₃OCO-D-Cha-Gly-Arg-pNA | Pefachrome FXa |
| Faktor XIa | H-D-Lys(Cbo)-Pro-Arg-pNA | Chromozym PCa |
| Faktor XIIa | H-D-HHT-Gly-Arg-pNA | Chromozym XII |
| Thrombin | CH₃SO₂-D-HHT-Gly-Arg-pNA | Pefachrome tPA |

| | | |
|---|---|---|
| Bz = Benzyl-, Lys(Cbo) = Omega-Carbobenzoxylysin, HHT = Hexahydrotyrosin, Cha = Cyclohexylalanin, pNA = para-Nitroanilin | | |

Für die Bestimmung wurden 200 µl Inhibitor-Lösung mit 25 µl Substrat gemischt und die Reaktion durch Zugabe von 50 µl Enzym gestartet. Nach einer Reaktionszeit von 3 - 5 min wurden 25 µl Essigsäure (50 %) zugegeben und die Extinktion bei 405 nm mit einem microplate reader (iEMS Reader MF 1401, Labsystems, Helsinki, Finnland) gemessen. Die Auswertung erfolgte graphisch entsprechend Dixon (Biochem. J., 55, 170, 1953).

Die für BAPA' bestimmten Kᵢ-Werte sind in Tabelle 3 dargestellt.

**Tabelle 3**

| Inhibitor | Kᵢ(µM) | | | | | |
|---|---|---|---|---|---|---|
| | Faktor Xa | Faktor VIIa | Faktor XIa | Faktor XIIa | PK | Thrombin |
| BAPA' | 0.0024 | 0.83 | 0.027 | 0.13 | 0.0083 | 0.0035 |

| | | | | | | |
|---|---|---|---|---|---|---|
| PK = Plasma-Kallikrein | | | | | | |

### Beispiel 8: Hemmung der Thrombin-Bildung in Hirudin-antikoaguliertem Plasma nach Aktivierung mittels Kaolin durch BAPA'

In diesem Beispiel wurde die Thrombin-Bildung in mit Hirudin antikoaguliertem Plasma durch Zugabe von Kaolin aktiviert und die Fähigkeit des Inhibitors BAPA', die Thrombin-Bildung zu inhibieren, untersucht.

Zunächst wurde von einem Patienten entnommenes Blut mit Hilfe des Thrombin-Inhibitors Hirudin antikoaguliert. Dazu wurden 9 Teile Blut mit 1 Teil Hirudin-Lösung (rekombinantes Hirudin HBW 023, Hoechst, Frankfurt, 2000 E/ml physiologischer NaCl-Lösung) gemischt und danach 10 Minuten bei 1300 Umdrehungen pro Minute (U/min) zentrifugiert. 900 µl des sich im Überstand befindlichen, mit Hirudin antikoagulierten Plasmas wurden in Polypropylen-Röhrchen mit 50 µl Inhibitorlösung bzw. 0,9 %iger NaCl-Lösung sowie 50 µl Kaolin-Suspension (aus APTT-Test von Roche Diagnostics, vor Gebrauch 1:250 verdünnt mit physiologischer NaCl-Lösung) versetzt und bei 37°C unter leichtem Schütteln inkubiert. Kaolin fungiert dabei als unphysiologische Oberfläche, die die Blutgerinnung aktiviert, wodurch die Thrombin-Bildung schließlich stimuliert wird. Zu bestimmten Zeitpunkten wurde die Menge des gebildeten Thrombins anhand der Bildung des Prothrombinfragments F1+2 immunologisch bestimmt (Enzygnost F1+2 micro, Dade Behring GmbH, Marburg). Diese Bestimmung basiert auf einem Enzymimmunoassay nach dem Sandwich-Prinzip. Dazu wurden zu bestimmten Zeitpunkten aliquote Volumina (600 µl) mit 0,1 M EDTA (40 µl) antikoaguliert und bei - 20 °C aufbewahrt. Zur Bestimmung der Konzentration des Prothrombinfragments F1+2 wurden 50 µl Plasma (gegebenenfalls verdünnt mit PBS, phosphate-buffered saline) mit monoklonalen Antikörpern gegen das humane Prothrombinfragment F1+2, die auf einer im Testkit enthaltenen Mikrotiterplatte fixiert waren, inkubiert. Nach einem Waschschritt mit PBS, der zur Entfernung von nicht von den Antikörpern gebundenem Probenmaterial diente, wurde mit Peroxidasekonjugierten Antikörpern gegen humanes Prothrombin, die an die freien F1+2-Determinanten binden, inkubiert. Nach einem weiteren Waschvorgang, bei dem die ungebundenen Enzym-konjugierten Antikörper entfernt werden, wurde Chromogen (o-Phenylendiamin/H₂O₂-Lösung) zugesetzt, um mit der an die Antikörper gegen humanes Prothrombin gebundene Peroxidase eine Farbreaktion zu erzeugen, deren Intensität proportional zur Konzentration des Prothrombinfragments F1+2 in der Probe ist. Diese wurde im Anschluss photometrisch bei einer Wellenlänge von 492 nm bestimmt.

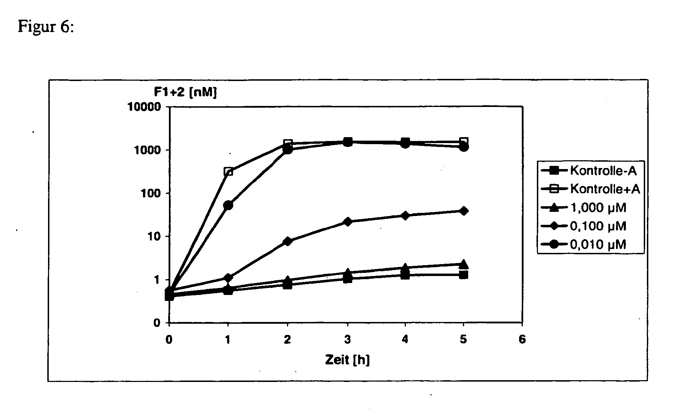

Wie aus der Figur 6 ersichtlich ist, ist BAPA' in der Lage, die durch die Aktivierung mit Kaolin stimulierte Thrombin-Bildung effektiv zu unterdrücken. BAPA', das den Blutgerinnungsfaktor Xa mit einem Kᵢ-Wert von 2,4 nM hemmt (Tabelle 3), ist noch bei einer Konzentration von 0,1 µM fähig, die Thrombin-Bildung nach Kaolin-Aktivierung > 97 % zu blockieren.

Bei der Interpretation der in diesem Beispiel erhaltenen Ergebnisse muss berücksichtigt werden, dass die Versuche in diesem Beispiel nicht in physiologischem Milieu durchgeführt wurden, sondern unter Verwendung von Kaolin, dass ein unphysiologischer, starker Aktivator der Thrombin-Bildung ist. Diese Ergebnisse zeigen daher eindrucksvoll, dass Blutplasma trotz der starken unphysiologischen Aktivierung der Thrombin-Bildung durch Kaolin mit Hilfe des erfindungsgemäßen Verfahrens unter Verwendung des erfindungsgemäßen Inhibitors erfolgreich antikoaguliert werden kann.

### Beispiel 9: Lagerung von humanem Venenblut nach Antikoagulation mit BAPA'

In diesem Experiment wurde untersucht, wie lange sich humanes Venenblut lagern lässt, ohne dass es dabei zur Bildung von Thrombin oder zur Aktivierung der Thrombozyten kommt.

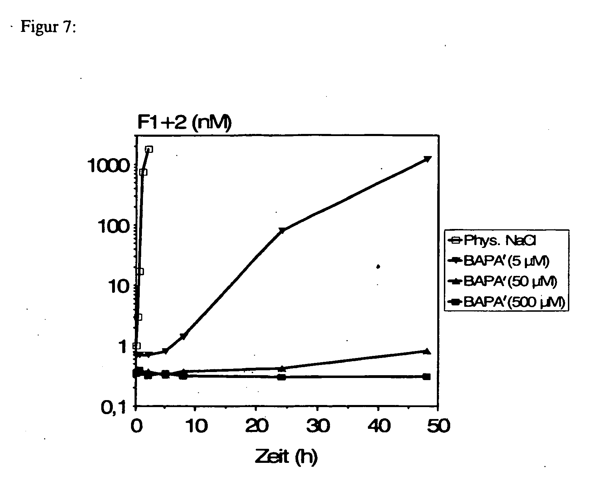

Dazu wurde von einem Patienten entnommenes Blut (9 Teile) mit Inhibitor-Lösung (0,05 mM, 0,5 mM bzw. 5 mM in physiologischer NaCl-Lösung; 1 Teil) gemischt und bei Zimmertemperatur (ca. 22± 2 °C) aufbewahrt. Die Menge des gebildeten Thrombins wurde im Verlauf der Lagerung anhand der Bildung des Prothrombinfragments F1+2 immunologisch ermittelt (Enzygnost F1+2 micro, Dade Behring GmbH, Marburg). Es konnte gezeigt werden, dass BAPA' in der Lage ist, antikoaguliertes Blut bei einer Konzentration im Blut von 500 bzw. 50 µM über mehr als 48 Stunden ungeronnen zu halten (Tabelle 4). Bei einer Konzentration von 5 µM verhindert BAPA' die Gerinnung für mehr als 8 Stunden.
Wie beispielhaft gezeigt wurde (Fig. 7), wird in dem jeweiligen Zeitraum weniger als 1 nM Thrombin (Nachweis über F1+2-Fragment) neu gebildet.

**Tabelle 4**

| Inhibitor | Konzentration im Blut (µM) | Dauer der Antikoagulation (h) |
|---|---|---|
| BAPA' | 500 | 48 |
| BAPA' | 50 | 48 |
| BAPA' | 5 | 8 |

### Beispiel 10: Induzierte Aktivierung der Thrombozyten nach der Antikoagulation mit BAPA'

In diesem Experiment wird gezeigt, dass sich die Thrombozyten in dem erfindungsgemäß antikoagulierten Blut durch Induktion problemlos aktivieren lassen, während dies bei mit Citrat antikoaguliertem Blut nicht über einen längeren Zeitraum der Fall ist.

Die nachstehend beschriebenen Untersuchungen wurden an 12 Frauen und 12 Männern durchgeführt, die innerhalb der letzten 14 Tage vor der Blutentnahme kein Aspirin oder andere Medikamente eingenommen hatten, die mit einer Beeinträchtigung der Thrombozytenfunktion einhergehen. Probanden mit anamnestischen Hinweisen auf eine erhöhte Blutungsneigung wurden ausgeschlossen. Es wurden jeweils zwei verschiedene Versuchsreihen durchgeführt, wobei in der einen Versuchsreihe die Antikoagulation mittels Zitrat und in der anderen Versuchsreihe mittels des erfindungsgemäßen Inhibitors BAPA' erfolgte.

Nach einer 30minütigen Ruhepause in sitzender Position wurde den Probanden innerhalb von 2 Stunden mit einer 21-gauge Butterfly-Nadel Blut aus der Kubitalvene entnommen, wobei möglichst wenig gestaut wurde. Das entnommene Blut wurde einerseits in Monovetten (Fa. Sarstedt) aspiriert, die 0,106 mol/l Trinatriumzitrat enthielten (Endverhältnis: 1 Teil Zitrat + 9 Teile Blut) und andererseits in Monovetten, die mit 500 µmol/l BAPA' vorbefüllt waren (Endverhältnis: 1 Teil BAPA' + 9 Teile Blut). Blut und Antikoagulans wurden sorgfältig durch leichtes wiederholtes Kippen der Röhrchen vermischt. Alle Proben wurden bei Raumtemperatur (22 ± 2 °C) gelagert. Die Analysen erfolgten nach 2, 4, 6, 8, 10 und 24 h.

### a) Turbidimetrische Plättchenaggregation (TPA)

Beim TPA-Assay wird mittels photometrischer Messung die Transmission von Licht durch die jeweilige Blutzellsuspension quantitativ bestimmt. Da PRP aufgrund der Lichtstreuung an den suspendierten, einzelnen Thrombozyten lediglich eine geringe Transmission für langwelliges Licht aufweist, PPP langwelliges Licht dagegen nahezu ungehindert passieren lässt, dient die relative prozentuale Transmission des Lichts durch die Probe als Maß für die Aggregation der Thrombozyten. Eine Aggregation der Thrombozyten hat eine Abnahme der Anzahl lichtstreuender Partikel zur Folge und lässt folglich die Transmission der Probe im Vergleich zu PRP mit nicht aggregierten Thrombozyten ansteigen.

Für die TPA-Assays wurde plättchenreiches und plättchenarmes Plasma (PRP bzw. PPP) hergestellt. Plättchenreiches Plasma wurde erhalten, indem das antikoagulierte Blut 2, 4, 6, 8, 10 und 24 h nach der Blutentnahme 10 Minuten bei 150 x g zentrifugiert und der Überstand anschließend abgenommen wurde. Plättchenarmes Plasma wurde durch 10-minütige Zentrifugation bei 3.500 g und Entnahme des Überstandes enthalten. Die Plättchenzahl wurde nicht eingestellt.

Für die TPA-Assays wurde die Thrombozyten-Aggregation in PRP durch Zugabe von Arachidonsäure (AA), ADP bzw. Collagen stimuliert. Dazu wurden 25 µl Arachidonsäure oder ADP zu 225 µl PRP (Endkonzentration von AA = 0,5 mmol/l bzw. von ADP = 5 µmol/l) bzw. 10 µl Kollagen aus Pferdeseren zu 240 µl PRP (Endkonzentration von Collagen = 2 µg/ml) gegeben.

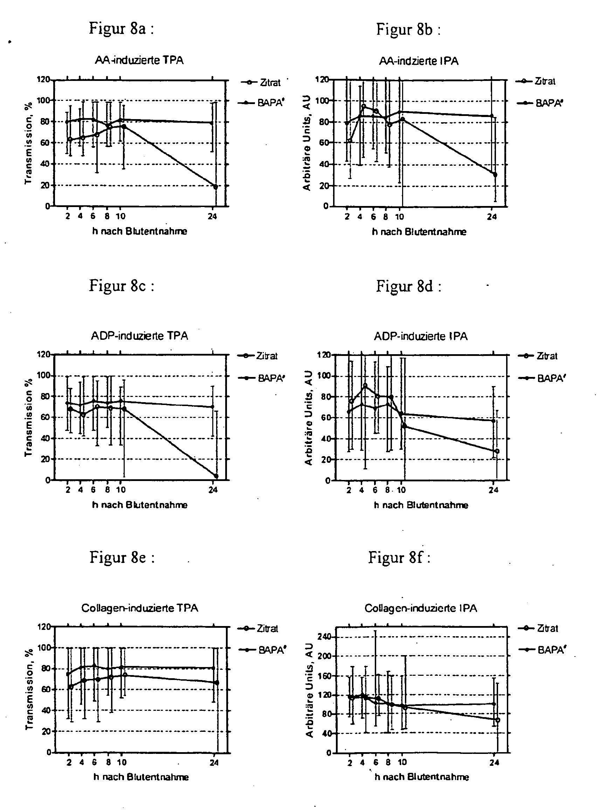

Die Aggregation der einzelnen Proben wurde mit Hilfe eines APACT 4 Aggregometers (Labitec, Arensburg) über 7 Minuten registriert. Als Referenz wurde die Differenz der Lichttransmission zwischen PRP und PPP auf einer linearen Skala gleich 100 % gesetzt. Anschließend wurde die durch die induzierte Plättchenaggregation hervorgerufene maximale Transmissionsänderung in der PRP-Probe registriert. Die Ergebnisse sind in Fig. 8 dargestellt.

### b) Impedanz-Vollblut-Plättchenaggregation (IPA)

Die Impedanz-Vollblut-Plättchenaggregometrie basiert auf der Messung der Änderung des elektrischen Widerstandes zwischen zwei Platinelektroden infolge der Anlagerung von Thrombozyten bei Stromfluss. Die Widerstandsänderung ist dabei proportional zur Thrombozytenaggregation.

Es wurden 300 µl antikoaguliertes Vollblut mit 300 µl physiologischer Kochsalzlösung verdünnt und 3 Minuten bei 37°C vorgewärmt. Danach wurden 20 µl der Agonisten zugegeben, um Endkonzentrationen von 0,5 mmol/l AA, 6,5 µmol/l ADP und 333,2 µg/ml Collagen zu erhalten. Die IPA-Assays erfolgten mit einem Multiplate Analyzer (Instrumentation Laboratory, Kirchheim). Die maximale Aggregation wurde 2, 4, 6, 8, 10 und 24 h nach der Blutentnahme jeweils über 6 Minuten registriert und in arbiträren Units (AU) ausgedrückt. Die Ergebnisse sind ebenfalls in Fig. 8 dargestellt.

### c) Partikelzahl-Plättchenaggregation (PPA)

Zur Partikelzahl-Plättchenaggregation wurde 2, 4, 6, 8, 10 und 24 h nach der Blutentnahme 1 ml antikoaguliertes Vollblut mit 1 ml 0,1 % (w/w) Glutaraldehyd in Saline-Phosphatpuffer, pH 7,4, versetzt und vorsichtig gemischt. In dieser Probe wurde die Ausgangs-Thrombozytenzahl bestimmt (ADVIA Analyzer, Bayer Vital, Fernwald). In ein zweites Röhrchen wurden 20 µl Agonist vorgelegt, um in 1 ml antikoaguliertem Vollblut Endkonzentrationen von z. B. 0,5 mmol/l AA, 20 µmol/l ADP bzw. 4 µg/ml Collagen zu erhalten. Anschließend wurden die Proben auf einen Blutbild-Agitator zum Durchmischen gelegt. Danach wurde jeweils 1 ml Glutaraldehyd-Puffer zugegeben, 3 Minuten gewartet, sorgfältig durchmischt und die Thrombozytenzahl bestimmt. Der Anteil aggregierter Thrombozyten in % wurde mit folgender Formel berechnet:
[(Thrombozytenzahl im Röhrchen ohne Agonist - Thrombozytenzahl im Röhrchen mit Agonist)/ Thrombozytenzahl im Röhrchen ohne Agonist]* 100.

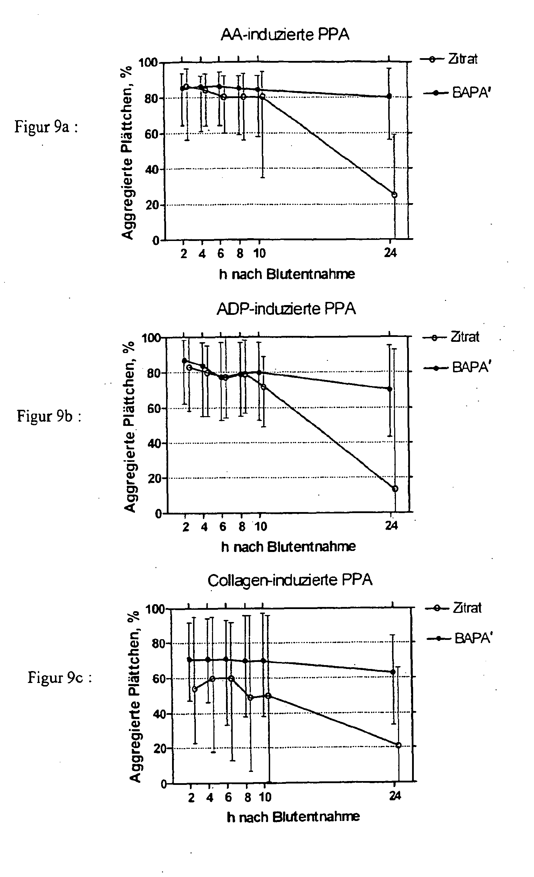

Die Ergebnisse sind in Fig. 9 dargestellt.

### d) Messung der Verschlusszeiten

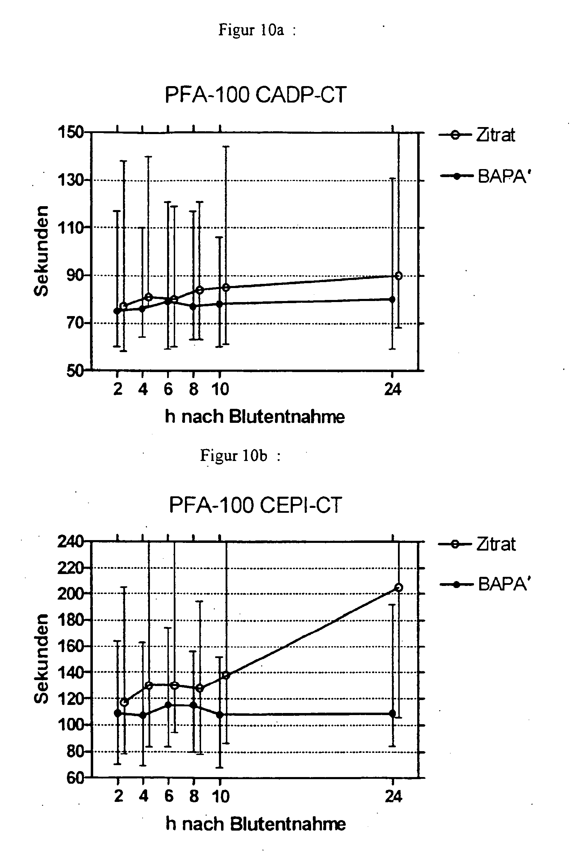

Die Messung der Verschlusszeiten (closure times, CT) wurde mit dem Platelet Function Analyzer 100 (PFA-100) durchgeführt. Dabei wurden Messzellen mit Membranen, die mit verschiedenen Agonisten beschichtet waren, mit antikoaguliertem Vollblut befüllt. Die Thrombozyten lagerten sich an den Membranen an, wo die Aggregation der Thrombozyten durch den Kontakt mit den Agonisten aktiviert wurde. Die dadurch induzierte Aggregation führte zu einer Verringerung des Blutflusses. Der PFA-100 ermittelte die Zeit bis zum vollständigen Membranverschluss (Verschlusszeit). Als Messzellen wurden die Collagen-Epinephrin (CEPI) und die Collagen-ADP (CADP) Cartridge verwendet. Die Ergebnisse sind in Fig. 10 dargestellt.

Die in Beispiel 10 durchgeführten Studien zeigen, dass sich durch die Antikoagulation mit dem erfindungsgemäßen Inhibitor gegenüber Zitrat eine wesentlich höhere Lagerungsstabilität der Proben hinsichtlich der Thrombozyten-Aggregabilität erreichen lässt. Mit dem erfindungsgemäßen Verfahren ist es somit möglich, Blutprodukte über einen langen Zeitraum haltbar zu machen und zu lagern, ohne dass es dabei zur Antikoagulation kommt, und ohne dass die Aktivierbarkeit der Thrombozyten dadurch beeinträchtigt wird.

### Beispiel 11: Haltbarmachung und Lagerung von Thrombozytapheresekonzentraten

In diesem Experiment wird gezeigt, dass sich Blutprodukte, wie zum Beispiel Thrombozytapheresekonzentrate unter Verwendung des erfindungsgemäßen Verfahrens zur Antikoagulation von humanem Blut ex vivo über einen langen Zeitraum haltbar machen und lagern lassen.

Ein Thrombozytapheresekonzentrat, das aus in Plasma suspendierten Thrombozyten besteht, wurde durch Thrombozytapherese mittels eines handelsüblichen Zellseparators unter Verwendung von zunächst Citrat als Antikoagulans hergestellt. In einem geschlossenen Thrombozytapherese-Beutelsystem wurde ein Satellitenbeutel integriert, der Calciumchlorid und einen erfindungsgemäßen direkten Inhibitor des Blugerinnungsfaktors Xa in physiologischer Lösung enthielt. Unmittelbar nach Beendigung der Thrombozytapherese wurde die Lösung im Satellitenbeutel dem Thrombozytapheresekonzentrat zugesetzt. Die Zugabe von Calciumionen (Recalcifizierung) stellte eine physiologische Calciumionenkonzentration im Thrombozytapheresekonzentrat ein, die zur Gewährleistung einer optimale Thrombozytenfunktion diente. Die Antikoagulation des nun wieder gerinnbaren Plasmas, in dem die Thrombozyten suspendiert waren, wurde durch die Zugabe des erfindungsgemäßen direkten Inhibitors des Blutgerinnungsfaktors Xa verhindert.

Das Thrombozytapheresekonzentrat wurde unter bei 22 ± 2°C unter ständiger Agitation gelagert. Nach 10-tägiger Lagerung besaß das Thrombozytapheresekonzentrat, das erfindungsgemäß mit dem direkten Inhibitor des Blutgerinnungsfaktors Xa antikoaguliert wurde, noch die gleiche Wirksamkeit wie konventionelle Thrombozytapheresekonzentrate nach 5 Tagen.

So zeigten die erfindungsgemäß antikoagulierten Thrombozytapheresekonzentrate auch nach 10-tägiger Lagerung noch das "Swirling-Phänomen" (das "Swirling-Phänomen" basiert auf der Lichtstreuung von bewegten, eine normale Morphologie aufweisenden Thrombozyten, und verschwindet, wenn die normalerweise scheibenförmigen Thrombozyten eine abnormale, kugelförmige Gestalt annehmen) und wiesen einen pH-Wert > 6,7 auf. Die Thrombozytenzahl war dabei immer größer als > 2 x 10¹¹/Einheit. Die Aggregation der Thrombozyten war durch Induktion mit 2 µg/ml Collagen auslösbar, wobei die Maximalamplitude 25 % betrug. Ferner konnte die Thrombozytenaggregation auch mit 5 bzw. 10 µmol/l ADP induziert werden.

Diese Ergebnisse zeigen ebenfalls, dass sich Blutprodukte, wie zum Beispiel Thrombozytapheresekonzentrate, durch das erfindungsgemäße Verfahren über einen langen Zeitraum haltbar machen und lagern lassen, ohne dass es dabei zur Antikoagulation kommt, und ohne dass die Aktivierbarkeit der Thrombozyten dadurch beeinträchtigt wird.

## Patentansprüche

1. Inhibitor des Blutgerinnungsfaktors Xa mit der Formel (I), 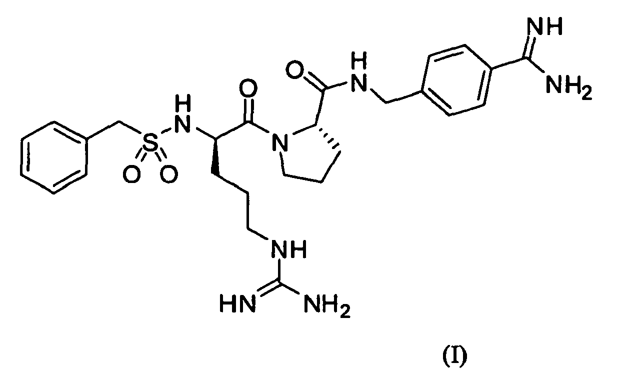 oder ein Salz mit Säuren davon.

2. Verfahren zur Antikoagulation von humanem Blut ex vivo, **dadurch gekennzeichnet, dass** dem entnommenen Blut der Inhibitor nach Anspruch 1 oder ein Salz mit Säuren davon zugesetzt wird.

3. Verfahren nach Anspruch 2, **dadurch gekennzeichnet, dass** der Inhibitor oder ein Salz mit Säuren davon in Lösung, vorzugsweise in physiologischer NaCl-Lösung oder einer Pufferlösung mit physiologischer Ionenstärke und/oder physiologischem pH-Wert vorliegt.

4. Verwendung des Inhibitors nach Anspruch 1 oder eines Salzes mit Säuren davon zur
a) ex vivo Diagnostik von zellulären Blutbestandteilen,
b) ex vivo Isolierung der zellulären Bestandteile des Blutes und/oder
c) Haltbarmachung und Lagerung von Blutprodukten, insbesondere von Thrombozytenkonzentraten oder Thrombozytapheresekonzentraten.

5. Verwendung nach Anspruch 4, wobei a) die Diagnose der Funktion der Thrombozyten, die Zählung, morphologische Beurteilung und Typisierung von Zellen im peripheren Blut und/oder die Bestimmung von Markem der aktivierten Thrombozyten zur Analyse von Hämostase und Fibrinolyse umfasst.

6. Röhrchen zur Entnahme von Blutproben, aufweisend einen Inhibitor nach Anspruch 1 oder ein Salz mit Säuren davon.

7. Aphereseschlauch-/beutelsystem, aufweisend wenigstens einen Auffangbeutel zur Aufnahme von Blut, **dadurch gekennzeichnet, dass** dieser Auffangbeutel eine wirksame Menge an dem Inhibitor nach Anspruch 1 oder ein Salz mit Säuren davon aufweist.

8. Thrombozytenkonzentrat in einem sterilen Auffangbeutel für die Infusion, aufweisend Inhibitor nach Anspruch 1 oder ein Salz mit Säuren davon.

9. Kit zur Untersuchung von zellulären Blutbestandteilen, umfassend Inhibitor des Blutgerinnungsfaktors Xa nach Anspruch 1 und ein Mittel zur Untersuchung von zellulären Blutbestandteilen.

10. Kit nach Anspruch 9, **dadurch gekennzeichnet, dass** der Kit für die Thrombozytenfunktionsdiagnosik bestimmt ist und das Mittel zur Untersuchung von zellulären Blutbestandteilen ein Mittel zur Thrombozytenfunktionsdiagnostik ist.

## Claims

1. Inhibitor of the blood coagulation factor Xa with the formula (I), 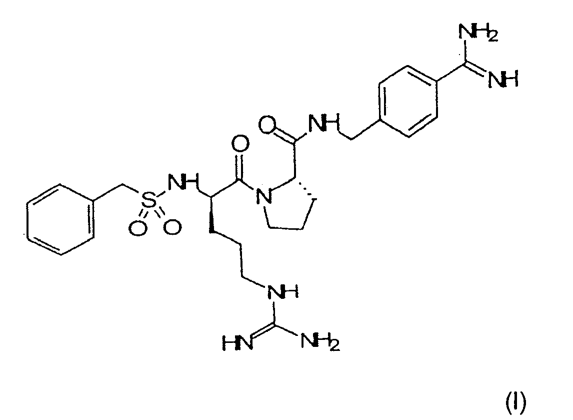 or a salt with acids thereof.

2. Method for anticoagulation of human blood ex vivo, **characterized in that** the inhibitor according to claim 1 or a salt with acids thereof is added to the collected blood.

3. Method according to claim 2, **characterized in that** the inhibitor or a salt with acids thereof is present in solution, preferably in physiological NaCl solution or in a buffer solution with physiological ionic strength and/or physiological pH-value.

4. Use of the inhibitor according to claim 1 or a salt with acids thereof, for
a) ex vivo diagnostic investigation of cellular blood components,
b) ex vivo isolation of cellular blood components and/or
c) preservation and storage of blood products, particularly of thrombocytes concentrates or thrombocytes aphaeresis concentrates.

5. Use according to claim 4, whereby a) comprises the diagnosis of thrombocytes function, counting, morphological assessment, and typing of cells in peripheral blood and/or determination of markers of the activated thrombocytes for analysis of haemostasis and fibrinolysis.

6. Tube for collection of blood samples containing an inhibitor according to claim 1 or a salt with acids thereof.

7. Aphaeresis tubing/bag system, having at least one bag for collection of blood, **characterized in that** this collection bag contains an effective quantity of inhibitor according to claim 1 or a salt with acids thereof.

8. Thrombocytes concentrate in a sterile collection bag for infusion, containing inhibitor according to claim 1 or a salt with acids thereof.

9. Kit for investigation of cellular blood components, comprising inhibitor of blood coagulation factor Xa according to claim 1 and an agent for evaluation of cellular blood components.

10. Kit according to claim 9, **characterized in that** the kit is intended for thrombocytes function diagnostic and **in that** the agent for evaluation of cellular blood components is an agent for thrombocytes function diagnosis.

## Revendications

1. Inhibiteur du facteur de coagulation sanguine Xa de formule (I), 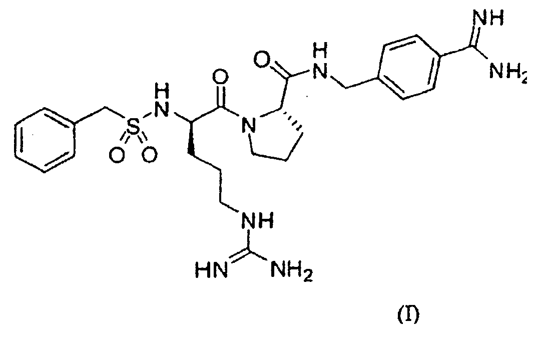 ou sel comprenant des acides correspondants.

2. Procédé d'anticoagulation ex vivo du sang humain, **caractérisé en ce que** l'inhibiteur selon la revendication 1 ou un sel comprenant des acides correspondants est ajouté au sang prélevé.

3. Procédé selon la revendication 2, **caractérisé en ce que** l'inhibiteur ou un sel comprenant des acides correspondants sont présentés en solution, de préférence en solution physiologique de NaCl ou dans une solution tampon à force ionique physiologique et/ou valeur de pH physiologique.

4. Utilisation d'un inhibiteur selon la revendication 1 ou d'un sel comprenant des acides correspondants pour
a) le diagnostic ex vivo de composants sanguins cellulaires,
b) l'isolement des composants cellulaires du sang et/ou
c) la conservation et le stockage de produits sanguins, en particulier de concentrés thrombocytaires ou de concentrés de thrombocytaphérèse.

5. Utilisation selon la revendication 4, où a) le diagnostic de fonction des thrombocytes comprend la numération, l'évaluation morphologique et le typage de cellules dans le sang périphérique et/ou la détermination de marqueurs des thrombocytes activés pour l'analyse de l'hémostase et de la fibrinolyse.

6. Tube pour le prélèvement d'échantillons sanguins, comportant un inhibiteur selon la revendication 1 ou un sel comprenant des acides correspondants.

7. Système de tuyau/poche d'aphérèse, comportant au moins une poche collectrice pour recueillir du sang, **caractérisé en ce que** ladite poche collectrice présente une teneur active d'un inhibiteur selon la revendication 1 ou d'un sel comprenant des acides correspondants.

8. Concentré thrombocytaire dans une poche collectrice stérile pour la perfusion, comportant un inhibiteur selon la revendication 1 ou un sel comprenant des acides correspondants.

9. Kit d'analyse de composants sanguins cellulaires, comprenant l'inhibiteur du facteur de coagulation sanguine Xa selon la revendication 1 et un agent d'analyse de composants sanguins cellulaires.

10. Kit selon la revendication 9, **caractérisé en ce que** le kit est destiné au diagnostic de fonction thrombocytaire et **en ce que** l'agent d'analyse de composants sanguins cellulaires est un agent de diagnostic de fonction thrombocytaire.
